# EUROPEAN PATENT SPECIFICATION

(11) **EP 3 268 740 B1**
(45) Date of publication and mention of the grant of the patent: **09.11.2022**
(21) Application number: 16762696.9
(22) Date of filing: 14.03.2016
(51) Int. Cl.: G01N 33/543, B01J 19/00

(54) **POLYPEPTIDE ARRAYS AND METHODS OF ATTACHING POLYPEPTIDES TO AN ARRAY**
POLYPEPTIDANORDNUNGEN UND VERFAHREN ZUR BINDUNG VON POLYPEPTIDEN AN EINE ANORDNUNG
PUCES À POLYPEPTIDES ET PROCÉDÉS DE FIXATION DE POLYPEPTIDES À UNE PUCE

(30) Priority: 12.03.2015 US 201562132405 P
(43) Date of publication of application: 17.01.2018
(60) Divisional application: 22199989.9
(73) Proprietor: Vibrant Holdings, LLC, San Carlos CA 94070 (US)
(72) Inventor: RAJASEKARAN, John, J., San Carlos, CA 94070 (US); WANG, Tianhao, San Carlos, CA 94070 (US); BEI, Kang, San Carlos, CA 94070 (US); JAYARAMAN, Vasanth, San Carlos, CA 94070 (US); KRISHNAMURTHY, Hari, Krishnan, San Carlos, CA 94070 (US)
(74) Representative: J A Kemp LLP
(86) International application number: PCT/US2016/022299
(87) International publication number: WO 2016/145434

(56) References cited:
- WO-A2-2014/052989
- WO-A2-2014/078606
- US-A1- 2005 042 771
- US-A1- 2008 305 964
- US-A1- 2014 349 888
- BALAKIREV MAXIM Y ET AL: "Photochemical patterning of biological molecules inside a glass capillary", ANALYTICAL CHEMISTRY, AMERICAN CHEMICAL SOCIETY, US, vol. 77, no. 17, 1 September 2005 (2005-09-01), pages 5474-5479, XP002356466, ISSN: 0003-2700, DOI: 10.1021/AC0504619
- Anonymous: "Molecular Probes (TM) Handbook A Guide to Fluorescent Probes and Labeling Technologies - 11th Edition Chapter 5 Crosslinking and Photoactivatable Reagents", , 1 January 2010 (2010-01-01), pages 1-18, XP055504756, Retrieved from the Internet: URL:https://www.thermofisher.com/content/d am/LifeTech/global/technical-reference-lib rary/Molecular%20Probes%20Handbook/chapter -pdfs/Ch-5-Crosslinking-Photoactivatable-R eagents.pdf?icid=WE216841 [retrieved on 2018-09-05]
- NAGA SIVA KUMAR GUNDA ET AL: "Micro-spot with integrated pillars (MSIP) for detection of dengue virus NS1", BIOMEDICAL MICRODEVICES, vol. 15, no. 6, 5 July 2013 (2013-07-05), pages 959-971, XP055504241, NL ISSN: 1387-2176, DOI: 10.1007/s10544-013-9787-3

## Description

### BACKGROUND

A typical microarray system is generally comprised of biomolecular probes, such as DNA, proteins, or peptides, formatted on a solid planar surface like glass, plastic, or silicon chip, plus the instruments needed to handle samples (automated robotics), to read the reporter molecules (scanners) and analyze the data (bioinformatic tools). Microarray technology can facilitate monitoring of many probes per square centimeter. Advantages of using multiple probes include, but are not limited to, speed, adaptability, comprehensiveness and the relatively cheaper cost of high volume manufacturing. The uses of such an array include, but are not limited to, diagnostic microbiology, including the detection and identification of pathogens, investigation of anti-microbial resistance, epidemiological strain typing, investigation of oncogenes, analysis of microbial infections using host genomic expression, and polymorphism profiles.

Recent advances in genomics have culminated in sequencing of entire genomes of several organisms, including humans. Genomics alone, however, cannot provide a complete understanding of cellular processes that are involved in disease, development, and other biological phenomena; because such processes are often directly mediated by polypeptides. Given that huge numbers of polypeptides are encoded by the genome of an organism, the development of high throughput technologies for analyzing polypeptides is of paramount importance.

Peptide arrays with distinct analyte-detecting regions or probes can be assembled on a single substrate by techniques well known to one skilled in the art. A variety of methods are available for creating a peptide microarray. These methods include: (a) chemo selective immobilization methods; and (b) in situ parallel synthesis methods which can be further divided into (1) SPOT synthesis and (2) photolithographic synthesis.

These methods are labor intensive and not suited for high throughput. These peptide arrays are expensive to manufacture, have low repeatability, may be unstable, require stringent storage conditions, take a long time to manufacture, and are limited in other ways. What is needed therefore, are improved peptide arrays and improved methods of fabricating peptide arrays.

Balakirev et al. (Analytical Chemistry, 2005, 77(17), 5474-5479) discloses a method for photochemical patterning of biological molecules onto the inner wall of fused-silica capillary. WO 2014/078606 A2 discloses substrates and arrays comprising a porous layer for synthesis and attachment of polymers or biomolecules.

### SUMMARY

Disclosed herein are formulations, substrates, and arrays. In certain embodiments, methods of attaching a biomolecule to an array using a photoactivated conjugation compound are disclosed. Methods of generating site-specific attachment of biomolecules to an array are also disclosed. Arrays generated by these methods and methods of using these arrays are also disclosed.

The invention as defined in the present claims provides:
(1) a method of attaching a polypeptide to a surface, comprising:
   obtaining a surface comprising a plurality of free amine groups attached to said surface;
   attaching a photoactivatable conjugation compound to said surface by contacting said surface with a conjugation solution comprising said photoactivatable conjugation compound, wherein said photoactivatable conjugation compound comprises an activated carboxylic acid group,
   and wherein said activated carboxylic acid group binds to the free amine groups attached to said surface;
   contacting said surface with a polypeptide; and
   selectively exposing said surface to electromagnetic radiation, wherein said electromagnetic radiation activates said attached photoactivatable conjugation compound and wherein said attached activated photoactivatable conjugation compound binds to said polypeptide, thereby attaching said polypeptide to said surface; and
(2) a method of attaching a polypeptide to a surface, comprising:
   obtaining a surface comprising a plurality of free carboxylic acid groups attached to said surface;
   contacting said surface with a carboxylic acid activation solution, thereby activating said carboxylic acid groups for binding to an amine group, wherein said carboxylic acid activation solution comprises a carbodiimide or N-hydroxysuccinimide or a compound selected from the group consisting of: 1-ethyl-3-(3-dimethylaminopropyl) carbodiimide, N,N'-diisopropylcarbodiimide, (Benzotriazol-1-yloxy)tripyrrolidinophosphonium hexafluorophosphate, bromo(tripyrrolidin-1-yl)phosphonium hexafluorophosphate, O-(7-azabenzotriazol-1-yl)-N,N,N',N'-tetramethyluronium hexafluorophosphate, O-Benzotriazole-N,N,N',N'-tetramethyl-uronium-hexafluoro-phosphate, and O-(Benzotriazol-1-yl)-N,N,N',N'-tetramethyluronium tetrafluoroborate,
   attaching a photoactivatable conjugation compound to said surface by contacting said surface with a conjugation solution comprising said photoactivatable conjugation compound, wherein said photoactivatable conjugation compound comprises an amine group, and wherein said amine group binds to the activated carboxylic acid group attached to said surface;
   contacting said surface with a polypeptide; and
   selectively exposing said surface to electromagnetic radiation, wherein said electromagnetic radiation activates said attached photoactivatable conjugation compound and wherein said attached activated photoactivatable conjugation compound bind to said polypeptide, thereby attaching said polypeptide to said surface.

The invention also provides a method as described by (1) or (2), wherein:
- said method further comprises washing said surface after attaching said photoactivatable conjugation compound to said surface;
- said activated photoactivatable conjugation compound binds to a polypeptide at a site selected from the group consisting of: a peptide backbone, a side group, an amine group, a carboxylic acid group;
- said contacting said surface with said conjugation solution comprises spin coating said conjugation solution onto said surface;
- said electromagnetic radiation has a wavelength of 248 nm; or a wavelength of 330-370 nm;
- said photoactivatable conjugation compound comprises a moiety selected from the group consisting of: a diazirine moiety, an aryl azide moiety, and a benzophenone moiety; or
- said conjugation solution comprises a polymer, and optionally wherein said polymer is polyvinyl pyrrollidone.

The invention also provides a method as described by (2), wherein:
- said method further comprises washing said surface after activating said carboxylic acid groups attached to said surface; or
- said photoactivatable conjugation compound comprises an N-hydroxysuccinimide moiety attached to an ester; or an N-hydroxysuccinimide ester functionally attached to a moiety selected from the group consisting of: a diazirine moiety, an aryl azide moiety, and a benzophenone moiety.

The invention also provides a method as described by (1) or (2), wherein:
- said polypeptide is a protein;
- said attachment of said polypeptide at said selectively exposed surfaces has an attachment efficiency of at least 90%, 91%, 92%, 93%, 94%, 95%, 96%, 97%, 98%, or 99%;
- said attachment of said polypeptides to said array has a high stability;
- said method is repeated to generate a surface comprising a plurality of unique polypeptides attached to said surface at selected locations; or
- said surface comprises at least 2, optionally at least 10, optionally at least 100, optionally at least 1,000, and optionally at least 10,000, unique polypeptides attached to said surface at selected locations.

### BRIEF DESCRIPTION OF THE SEVERAL VIEWS OF THE DRAWINGS

These and other features, aspects, and advantages of the present invention will become better understood with regard to the following description, and accompanying drawings, where:
**Figure 1** shows synthesis of a substrate comprising pillars.
**Figure 2** shows attachment of a first protein to an amine derivatized array using a conjugation compound.
**Figure 3** shows attachment of a second protein to an amine derivatized array using a conjugation compound.
**Figure 4** shows attachment of a first protein to a carboxylic acid derivatized array using a conjugation compound.
**Figure 5** shows attachment of a second protein to a carboxylic acid derivatized array using a conjugation compound.
**Figure 6** shows synthesis of a substrate comprising pillars having a hydroxylated top surface.
**Figure 7** shows a measure of fluorescence of binding to anti-IL-6 and anti-TNF alpha antibodies binding to IL-6 and TNF alpha proteins attached to a substrate via conjugation groups.

### DETAILED DESCRIPTION

Terms used in the claims and specification are defined as set forth below unless otherwise specified.

As used herein the term "wafer" refers to a slice of semiconductor material, such as a silicon or a germanium crystal generally used in the fabrication of integrated circuits. Wafers can be in a variety of sizes from, *e.g.,* 25.4 mm (1 inch) to 300 mm (11.8 inches) along one dimension with thickness from, *e.g*., 275µm to 775µm.

As used herein the term "photoresist" or "resist" or "photoactive material" or refers to a light-sensitive material that changes its solubility in a solution when exposed to ultra violet or deep ultra violet radiation. Photoresists are organic or inorganic compounds that are typically divided into two types: positive resists and negative resists. A positive resist is a type of photoresist in which the portion of the photoresist that is exposed to light becomes soluble to the photoresist developer. The portion of the photoresist that is unexposed remains insoluble to the photoresist developer. A negative resist is a type of photoresist in which the portion of the photoresist that is exposed to light becomes insoluble to the photoresist developer. The unexposed portion of the photoresist is dissolved by the photoresist developer.

As used herein the term "photomask" or "reticle" or "mask" refers to an opaque plate with transparent patterns or holes that allow light to pass through. In a typical exposing process, the pattern on a photomask is transferred onto a photoresist. The photomask or reticle or mask is used to generate a pattern of electromagnetic radiation exposure, thus allowing site specific activation of, e.g., photoactive compounds or photoactivatable conjugation groups.

As used herein the term "photoactive compound" refers to compounds that are modified when exposed to electromagnetic radiation. These compounds include, for example, cationic photoinitiators such as photoacid or photobase generators, which generate an acid or a base, respectively, when exposed to electromagnetic radiation. A photoinitiator is a compound especially added to a formulation to convert electromagnetic radiation into chemical energy in the form of initiating species, *e.g.,* free radicals or cations. The acid, base, or other product of a photoactive compound exposed to electromagnetic radiation may then react with another compound in a chain reaction to produce a desired chemical reaction. The spatial orientation of the occurrence of these chemical reactions is thus defined according to the pattern of electromagnetic radiation the solution or surface comprising photoactive compounds is exposed to. This pattern may be defined, *e.g.,* by a photomask or reticle.

As used herein, the term conjugation compound refers to a compound that binds to functional groups on a substrate and is capable of being activated to bind to a biomolecule, thus attaching the biomolecule to the substrate. A photoactivatable conjugation compound or photoactive conjugation compound refers to a compound that is activated to conjugate to a biomolecule when exposed to electromagnetic radiation. These compounds include, for example, compounds comprising a diazirine moiety, an aryal azide moiety, or a benzophenone moiety.

As used herein the term "coupling molecule" or "monomer molecule" includes any natural or artificially synthesized amino acid with its amino group protected with a fluorenylmethyloxycarbonyl group or a t-butoxycarbonyl group. These amino acids may have their side chains protected as an option. Examples of coupling molecules include Boc-Gly-Oh, Fmoc- Trp-Oh. Other examples are described below.

As used here in the term "coupling" or "coupling process" or "coupling step" refers to a process of forming a bond between two or more molecules such as a linking molecule or a coupling molecule. A bond can be a covalent bond such as a peptide bond. A peptide bond can a chemical bond formed between two molecules when the carboxyl group of one coupling molecule reacts with the amino group of the other coupling molecule, releasing a molecule of water (H₂O). This is a dehydration synthesis reaction (also known as a condensation reaction), and usually occurs between amino acids. The resulting CO-NH bond is called a peptide bond, and the resulting molecule is an amide.

As used herein the term "coupling efficiency" refers to the probability of successful addition of a monomer to a reaction site (*e*.*g*., at the end of a polymer) available for binding to the monomer. For example, during the growth of a peptide chain in the N to C orientation, a polypeptide having a free carboxyl group would bind to a peptide having a free amine group under appropriate conditions. The coupling efficiency gives the probability of the addition of a free peptide to the free carboxyl group under certain conditions. It may be determined in bulk, *e.g.,* by monitoring single monomer additions to several unique reaction sites simultaneously.

As used herein the terms "bio molecule," "polypeptide," "peptide," or "protein" are used interchangeably to describe a chain or polymer of amino acids that are linked together by bonds. Accordingly, the term "peptide" as used herein includes a dipeptide, tripeptide, oligopeptide, and polypeptide. The term "peptide" is not limited to any particular number of amino acids. In some aspects, a peptide contains about 2 to about 50 amino acids, about 5 to about 40 amino acids, or about 5 to about 20 amino acids. A molecule, such as a protein or polypeptide, including an enzyme, can be a "native" or "wild-type" molecule, meaning that it occurs naturally in nature; or it may be a "mutant," "variant," "derivative," or "modification," meaning that it has been made, altered, derived, or is in some way different or changed from a native molecule or from another molecule such as a mutant.

As used herein the term "linker molecule" or "spacer molecule" includes any molecule that does not add any functionality to the resulting peptide but spaces and extends out the peptide from the substrate, thus increasing the distance between the substrate surface and the growing peptide. This generally reduces steric hindrance with the substrate for reactions involving the peptide (including uni-molecular folding reactions and multimolecular binding reactions) and so improves performance of assays measuring one or more aspects of peptide functionality.

As used herein the term "developer" refers to a solution that can selectively dissolve the materials that are either exposed or not exposed to light. Typically developers are water-based solutions with minute quantities of a base added. Examples include tetramethyl ammonium hydroxide in water-based developers. Developers are used for the initial pattern definition where a commercial photoresist is used. Use of developers are described in Example 1 below.

As used herein the term "protecting group" includes a group that is introduced into a molecule by chemical modification of a functional group in order to obtain chemoselectivity in a subsequent chemical reaction. Chemoselectivity refers to directing a chemical reaction along a desired path to obtain a pre-selected product as compared to another. For example, the use of tboc as a protecting group enables chemoselectivity for peptide synthesis using a light mask and a photoacid generator to selectively remove the protecting group and direct pre-determined peptide coupling reactions to occur at locations defined by the light mask.

As used herein the term "microarrays" refers to a substrate on which different probe molecules of protein or specific DNA binding sequences have been affixed at separate locations in an ordered manner thus forming a microscopic array.

As used herein the term "microarray system" refers to a system usually comprised of bio molecular probes formatted on a solid planar surface like glass, plastic or silicon chip plus the instruments needed to handle samples (automated robotics), to read the reporter molecules (scanners) and analyze the data (bioinformatic tools).

As used herein the term "patterned region" or "pattern" or "location" refers to a region on the substrate on which are grown different features. These patterns can be defined using photomasks.

As used herein the term "derivatization" refers to the process of chemically modifying a surface to make it suitable for bio molecular synthesis. Typically derivatization includes the following steps: making the substrate hydrophilic, adding an amino silane group, and attaching a linker molecule.

As used herein the term "capping" or "capping process" or "capping step" refers to the addition of a molecule that prevents the further reaction of the molecule to which it is attached. For example, to prevent the further formation of a peptide bond, the amino groups are typically capped with an acetic anhydride molecule. In other embodiments, ethanolamine is used.

As used herein the term "diffusion" refers to the spread of photoacid through random motion from regions of higher concentration to regions of lower concentration.

As used herein the term "dye molecule" refers to a dye which typically is a colored substance that can bind to a substrate. Dye molecules can be useful in detecting binding between a feature on an array and a molecule of interest.

As used herein, the terms "immunological binding" and "immunological binding properties" refer to the non-covalent interactions of the type which occur between an immunoglobulin molecule and an antigen for which the immunoglobulin is specific.

As used herein the term "biological sample" refers to a sample derived from biological tissue or fluid that can be assayed for an analyte(s) of interest. Such samples include, but are not limited to, sputum, amniotic fluid, blood, blood cells (*e.g.,* white cells), tissue or fine needle biopsy samples, urine, peritoneal fluid, and pleural fluid, or cells therefrom. Biological samples may also include sections of tissues such as frozen sections taken for histological purposes. Although the sample is typically taken from a human patient, the assays can be used to detect analyte(s) of interest in samples from any organism (*e.g*., mammal, bacteria, virus, algae, or yeast) or mammal, such as dogs, cats, sheep, cattle, and pigs. The sample may be pretreated as necessary by dilution in an appropriate buffer solution or concentrated, if desired.

As used herein, the term "assay" refers to a type of biochemical test that measures the presence or concentration of a substance of interest in solutions that can contain a complex mixture of substances.

The term "antigen" as used herein refers to a molecule that triggers an immune response by the immune system of a subject, e.g., the production of an antibody by the immune system. Antigens can be exogenous, endogenous or auto antigens. Exogenous antigens are those that have entered the body from outside through inhalation, ingestion or injection. Endogenous antigens are those that have been generated within previously-normal cells as a result of normal cell metabolism, or because of viral or intracellular bacterial infection. Auto antigens are those that are normal protein or protein complex present in the host body but can stimulate an immune response.

As used herein the term "epitope" or "immunoactive regions" refers to distinct molecular surface features of an antigen capable of being bound by component of the adaptive immune system, *e.g*., an antibody or T cell receptor. Antigenic molecules can present several surface features that can act as points of interaction for specific antibodies. Any such distinct molecular feature can constitute an epitope. Therefore, antigens have the potential to be bound by several distinct antibodies, each of which is specific to a particular epitope.

As used herein the term "antibody" or "immunoglobulin molecule" refers to a molecule naturally secreted by a particular type of cells of the immune system: B cells. There are five different, naturally occurring isotypes of antibodies, namely: IgA, IgM, IgG, IgD, and IgE.

As used herein, the term "activated carboxylic acid group" refers to a carboxylic acid group that has a leaving group bound such that it will readily bind to an amine group. In some embodiments, a carbodiimide or N-hydroxysuccinimide activates a carboxylic acid group to increase its probability of binding to an amine group. In some embodiments, an activated carboxylic acid group refers to an ester or carbonyl bound to a group that will be removed upon interaction with an amine group, thus resulting in covalent bond formation between the ester or carbonyl group and the amine group.

It must be noted that, as used in the specification and the appended claims, the singular forms "a," "an," and "the" include plural referents unless the context clearly dictates otherwise.

### Compositions

### Formulations

Disclosed herein are formulations such as photoactive conjugation solutions and polypeptide formulations. These formulations can be useful in the manufacture and/or use of, e.g., substrates and/or polypeptide arrays disclosed herein.

### Photoactive Conjugation Solutions

Disclosed herein are photoactive conjugation solutions. In some aspects, a photoactive conjugation solution can include components such as a solvent, a photoactive conjugation compound, and a polymer.

In one aspect, a photoactive conjugation solution can include a photoactive conjugation compound (i.e., a conjugation compound). A photoactive conjugation compound comprises a chemically inert moiety that becomes reactive when exposed to ultraviolet or visible light. Exposure of the photoactive conjugation compounds to electromagnetic radiation is a primary photochemical event that produces a compound that binds to a polypeptide. A photoactive conjugation solution may comprise a photoactive conjugation compound comprising a radiation-sensitive binding precursor comprising a chemical group that can react by elimination, addition, or rearrangement; and optional additives to improve performance or processability.

In some aspects, a photoactive coupling formulation includes a photoactive conjugation compound in a polymer matrix dispersed in a solvent. In some aspects, the polymer in the composition of the photoresist is generally inert and non-crosslinking.

In some aspects, a photoactive compound can have an amine group or a carboxylic acid group. In some embodiments, the carboxylic acid group is activated by binding to a strong leaving group to induce a covalent bond with an amine group. In some embodiments, the amine group is used to bind to a carboxylic acid group attached to the surface of an array. The photoactive compound comprises a photoactive moiety to convert absorbed light energy, UV or visible light, into chemical energy in the form of initiating species, *e.g.,* free radicals or cations.

In one embodiment, photoactive conjugation compounds are used as heterobifunctional crosslinkers to attach a polypeptide to an array surface. In one embodiment, the photoactive conjugation compounds further comprise an amine group to bind to a carboxylic acid group attached to an array surface. In another embodiment, the photoactive conjugation compound further comprises a carboxylic acid group which is activated to bind to an amine group attached to an array surface. Once bound to the array surface, the photoactive conjugation group is site-specifically activated to conjugate a desired polypeptide, protein or other biomolecule.

In some aspects, a photoactive compound comprises an aryl azide, a diazirine, or a benzophenone moiety. Aryl azides (also called phenylazides) form a nitrene group when exposed to UV light. The nitrene group can initiate addition reactions with double bonds or insertion into C-H and N-H sites or can undergo ring expansion to react with a nucleophile (e.g., primary amine). Reactions can be performed in a variety of amine-free buffer conditions to conjugate proteins or even molecules devoid of the usual functional group "handles". The diazirine (azipentanoate) moiety has better photostability than phenyl azide groups, and it is more easily and efficiently activated with long-wave UV light (330-370 nm). Photoactivation of diazirine creates reactive carbene intermediates. Such intermediates can form covalent bonds through addition reactions with any amino acid side chain or peptide backbone at distances corresponding to the spacer arm lengths of the particular reagent. Diazirine-analogs of amino acids can be incorporated into protein structures by translation, enabling specific recombinant proteins to be activated as the crosslinker.

In some embodiments, the conjugation solution comprises a conjugation compound, a solvent, and a polymer. In one embodiment, the conjugation compound is an NHS ester of aryl azide, diazirine, or benzophenone. In another embodiment, the conjugation compound is an amine group functionally linked to an aryl azide, a diazirine, or a benzophenone. In some aspects, the carbodiimide precursor is present in the activation solution at a concentration of 2.5% by weight. In some aspects the conjugation compound is present in the conjugation solution at a concentration of 0.1, 0.2, 0.3, 0.4, 0.5, 0.6, 0.7, 0.8, 0.9, 1.0, 1.1, 1.2, 1.3., 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, or 5.0 weight % of the total formulation concentration.

In some aspects, a polymer is a non-crosslinking inert polymer. In some aspects, a polymer is a polyvinyl pyrrolidone. The general structure of polyvinyl pyrrolidone is as follows, where n is any positive integer greater than 1.:

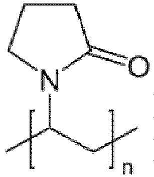

In some aspects, a polymer is a polymer of vinyl pyrrolidone. In some aspects, a polymer is polyvinyl pyrrolidone. Poly vinyl pyrrollidone is soluble in water and other polar solvents. When dry it is a light flaky powder, which generally readily absorbs up to 40% of its weight in atmospheric water. In solution, it has excellent wetting properties and readily forms films. In some aspects, a polymer is a vinyl pyrrolidone or a vinyl alcohol. In some aspects, a polymer is a polymethyl methacrylate.

In some aspects, a polymer is 2.5-5 weight % of the total formulation concentration. In some aspects, a polymer is about 0.5-5 weight % of the total formulation concentration. In some aspects, a polymer is about less than 0.1, 0.1, 0.2, 0.3, 0.4, 0.5, 0.6, 0.7, 0.8, 0.9, 1.0, 1.1, 1.2, 1.3., 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, or greater than 5.0 weight % of the total formulation concentration.

In some aspects, a solvent is water, ethyl lactate, n methyl pyrrollidone or a combination thereof. In some aspects, ethyl lactate can be dissolved in water to more than 50% to form a solvent. In some aspects, a solvent can be about 10% propylene glycol methyl ether acetate (PGMEA) and about 90% DI water. In some aspects, a solvent can include up to about 20% PGMEA. In some aspects, a solvent can include 50% ethyl lactate and 50% n methyl pyrrollidone. In some aspects, a solvent is n methyl pyrrollidone. In some aspects, a solvent is water, an organic solvent, or combination thereof. In some aspects, the organic solvent is N Methyl pyrrolidone, di methyl formamide or combinations thereof.

In some aspects, the solvent is about 80-90 weight % of the total formulation concentration. In some aspects, the solvent is about less than 70, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or greater than 99 weight % of the total formulation concentration.

### Carboxylic Acid Activating Formulations

Disclosed herein are activation formulations for activating carboxylic acid so that it reacts with a free amine group of a biomolecule, e.g., conjugation compound. An activation formulation can include components such as a carboxylic acid group activating compound and a solvent. In one embodiment, the carboxylic acid group activating compound is a carbodiimide or a carbodiimide precursor. In some aspects, the carbodiimide is 1-ethyl-3-(3-dimethylaminopropyl) carbodiimide. In some embodiments, the carboxylic acid group activating compound is *N*-Hydroxysuccinimide (NHS). In some embodiments, the solvent is water. In some embodiments, the carboxylic acid group activating compound converts the carboxylic acid to a carbonyl group (i.e., carboxylic acid group activation). In some embodiments, the carboxylic acid group is activated for 5, 10, 15, 20, 30, 45, or 60 minutes after exposure to an activation formulation.

In some aspects, the activation formulation comprises 4 % by weight of 1-ethyl-3-(3-dimethylaminopropyl) carbodiimide and 2 % by weight of N-hydroxysuccinimide (NHS) were dissolved in deionized water.

In some embodiments, the carboxylic acid group activating compound is a carbodiimide precursor. In one aspect, the carbodiimide precursor is converted to a carbodiimide through exposure to radiation, e.g., ultraviolet radiation. In one embodiment, the carbodiimide precursor is a thione. The carbodiimide precursor may also be referred to as a photoactivated carbodiimide. In one embodiment, photoactivated carbodiimides are used to provide site-specific activation of carboxylic acid groups on an array by spatially controlling exposure of the photoactivated carbodiimide solution to electromagnetic radiation at a preferred activation wavelength. In some embodiments, the preferred activation wavelength is 248 nm.

In one embodiment, the carbodiimide precursor is a thione that is_converted to carbodiimide via photoactivation. In one aspect, the thione is converted to a hydroxymethyl phenyl carbodiimide after exposure to electromagnetic radiation. In some embodiments, the thione is 4,5-dihydro-4-(hydroxymethyl)-1-phenyl-1H-tetrazole-5-thione, 1-ethyl-4-dimethylaminopropyl tetrazole 5-thione, 1,3-Bis(2,2-dimethyl-1,3-dioxolan-4-ylmethyl)-5-thione, 4-cyclohexyl -1H-tetrazole-5(4H)-thione, or 1-phenyl-4-(piperidinomethyl) tetrazole-5(4H)-thione.

In some embodiments, the activation solution comprises a carbodiimide precursor, a solvent, and a polymer. In one embodiment, the carbodiimide precursor is 4,5-dihydro-4-(hydroxymethyl)-1-phenyl-1H-tetrazole-5-thione, 1-ethyl-4-dimethylaminopropyl tetrazole 5-thione, or 1,3-Bis(2,2-dimethyl-1,3-dioxolan-4-ylmethyl)-5-thione. In some aspects, the carbodiimide precursor is present in the activation solution at a concentration of 2.5% by weight. In some aspects the carbodiimide precursor is present in the activation solution at a concentration of 0.1, 0.2, 0.3, 0.4, 0.5, 0.6, 0.7, 0.8, 0.9, 1.0, 1.1, 1.2, 1.3., 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, or 5.0 weight % of the total formulation concentration.

In some embodiments, the solvent is water. In some aspects, the solvent is about 80-90 weight % of the total formulation concentration. In some aspects, the solvent is about less than 70, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or greater than 99 weight % of the total formulation concentration.

In some aspects, a polymer is a polyvinyl pyrrolidone and/or a polyvinyl alcohol. In some aspects, a polymer is about 0.5-5 weight % of the total formulation concentration. In some aspects, a polymer is about less than 0.1, 0.1, 0.2, 0.3, 0.4, 0.5, 0.6, 0.7, 0.8, 0.9, 1.0, 1.1, 1.2, 1.3., 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, or greater than 5.0 weight % of the total formulation concentration.

In some aspects, a coupling reagent is a carbodimide. In some aspects, a coupling reagent is a triazole. In some aspects, a coupling reagent is 1-ethyl-3-(3-dimethylaminopropyl) carbodiimide. In some aspects, a coupling reagent is about 0.5-5 weight % of the total formulation concentration. In some aspects, a coupling reagent is about less than 0.1, 0.1, 0.2, 0.3, 0.4, 0.5, 0.6, 0.7, 0.8, 0.9, 1.0, 1.1, 1.2, 1.3., 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, or greater than 5.0 weight % of the total formulation concentration.

### Linker Formulations

Also disclosed herein is a linker formulation. A linker formulation can include components such as a solvent, a polymer, a linker molecule, and a coupling reagent. In some aspects, the polymer is 1 weight % polyvinyl alcohol and 2.5 weight % poly vinyl pyrrollidone, the linker molecule is 1.25 weight % polyethylene oxide, the coupling reagent is 1 weight % 1-ethyl-3-(3-dimethylaminopropyl) carbodiimide, and the solvent includes water. In some aspects, the polymer is 0.5-5 weight % polyvinyl alcohol and 0.5-5 weight % poly vinyl pyrrollidone, the linker molecule is 0.5-5 weight % polyethylene oxide, the coupling reagent is 0.5-5 weight % 1-ethyl-3-(3-dimethylaminopropyl) carbodiimide, and the solvent includes water.

In some aspects, the solvent is water, an organic solvent, or a combination thereof. In some aspects, the organic solvent is N Methyl pyrrolidone, Di methyl formamide, Di chloromethane, Di methyl sulfoxide, or a combination thereof. In some aspects, the solvent is about 80-90 weight % of the total formulation concentration. In some aspects, the solvent is about less than 70, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or greater than 99 weight % of the total formulation concentration.

In some aspects, a polymer is a polyvinyl pyrrolidone and/or a polyvinyl alcohol. The general structure of polyvinyl alcohol is as follows, where n is any positive integer greater than 1:

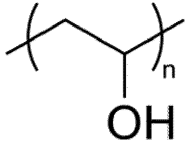

In some aspects, a polymer is about 0.5-5 weight % of the total formulation concentration. In some aspects, a polymer is about less than 0.1, 0.1, 0.2, 0.3, 0.4, 0.5, 0.6, 0.7, 0.8, 0.9, 1.0, 1.1, 1.2, 1.3., 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, or greater than 5.0 weight % of the total formulation concentration.

A linker molecule can be a molecule inserted between a surface disclosed herein and peptide that is being synthesized via a coupling molecule. A linker molecule does not necessarily convey functionality to the resulting peptide, such as molecular recognition functionality, but can instead elongate the distance between the surface and the peptide to enhance the exposure of the peptide's functionality region(s) on the surface. In some aspects, a linker can be about 4 to about 40 atoms long to provide exposure. The linker molecules can be, for example, aryl acetylene, ethylene glycol oligomers containing 2-10 monomer units (PEGs), diamines, diacids, amino acids, and combinations thereof. Examples of diamines include ethylene diamine and diamino propane. Alternatively, linkers can be the same molecule type as that being synthesized (*e.g*., nascent polymers or various coupling molecules), such as polypeptides and polymers of amino acid derivatives such as for example, amino hexanoic acids. In some aspects, a linker molecule is a molecule having a carboxylic group at a first end of the molecule and a protecting group at a second end of the molecule. In some aspects, the protecting group is a t-Boc protecting group or an F-Moc protecting group. In some aspects, a linker molecule is or includes an aryl acetylene, a polyethyleneglycol, a nascent polypeptide, a diamine, a diacid, a peptide, or combinations thereof. In some aspects, a linker molecule is about 0.5-5 weight % of the total formulation concentration. In some aspects, a linker molecule is about less than 0.1, 0.1, 0.2, 0.3, 0.4, 0.5, 0.6, 0.7, 0.8, 0.9, 1.0, 1.1, 1.2, 1.3., 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, or greater than 5.0 weight % of the total formulation concentration.

The unbound portion of a linker molecule, or free end of the linker molecule, can have a reactive functional group which is blocked, protected, or otherwise made unavailable for reaction by a removable protective group, *e.g.,* t-Boc or F-Moc as noted above. The protecting group can be bound to a monomer, a polymer, or a linker molecule to protect a reactive functionality on the monomer, polymer, or linker molecule. Protective groups that can be used include all acid and base labile protecting groups. For example, peptide amine groups can be protected by t-butoxycarbonyl (t-BOC or BOC) or benzyloxycarbonyl (CBZ), both of which are acid labile, or by 9-fluorenylmethoxycarbonyl (FMOC), which is base labile.

Additional protecting groups that can be used include acid labile groups for protecting amino moieties: tert-amyloxycarbonyl, adamantyloxycarbonyl, 1-methylcyclobutyloxycarbonyl, 2-(p-biphenyl)propyl(2)oxycarbonyl, 2-(p-phenylazophenylyl)propyl(2)oxycarbonyl, alpha,alpha-dimethyl-3,5-dimethyloxybenzyloxy-carbonyl, 2-phenylpropyl(2)oxycarbonyl, 4-methyloxybenzyloxycarbonyl, furfuryloxycarbonyl, triphenylmethyl (trityl), p-toluenesulfenylaminocarbonyl, dimethylphosphinothioyl, diphenylphosphinothioyl, 2-benzoyl-1-methylvinyl, o-nitrophenylsulfenyl, and 1-naphthylidene; as base labile groups for protecting amino moieties: 9 fluorenylmethyloxycarbonyl, methylsulfonylethyloxycarbonyl, and 5-benzisoazolylmethyleneoxycarbonyl; as groups for protecting amino moieties that are labile when reduced: dithiasuccinoyl, p-toluene sulfonyl, and piperidino-oxycarbonyl; as groups for protecting amino moieties that are labile when oxidized: (ethylthio)carbonyl; as groups for protecting amino moieties that are labile to miscellaneous reagents, the appropriate agent is listed in parenthesis after the group: phthaloyl (hydrazine), trifluoroacetyl (piperidine), and chloroacetyl (2-aminothiophenol); acid labile groups for protecting carboxylic acids: tert-butyl ester; acid labile groups for protecting hydroxyl groups: dimethyltrityl. (See also, Greene, T. W., Protective Groups in Organic Synthesis, Wiley-Interscience, NY, (1981)).

### Substrates

Also disclosed herein are substrates. In some aspects a substrate surface is planar (i.e., 2-dimensional). In some aspects, a substrate can include a porous layer (i.e., a 3-dimensional layer) comprising functional groups for binding a first monomer building block. In some aspects, a substrate surface comprises pillars for peptide attachment or synthesis. In some embodiments, a porous layer is added to the top of the pillars.

### Porous Layer Substrates

Porous layers which can be used are flat, permeable, polymeric materials of porous structure which have a carboxylic acid functional group (which is native to the constituent polymer or which is introduced to the porous layer) for attachment of the first peptide building block. For example, a porous layer can be comprised of porous silicon with functional groups for attachment of a polymer building block attached to the surface of the porous silicon. In another example, a porous layer may comprise a cross-linked polymeric material. In some embodiments, the porous layer may employ polystyrenes, saccharose, dextrans, polyacryloylmorpholine, polyacrylates, polymethylacrylates, polyacrylamides, polyacrylolpyrrolidone, polyvinylacetates, polyethyleneglycol, agaroses, sepharose, other conventional chromatography type materials and derivatives and mixtures thereof. In some embodiments, the porous layer building material is selected from: poly(vinyl alcohol), dextran, sodium alginate, poly(aspartic acid), poly(ethylene glycol), poly(ethylene oxide), poly(vinyl pyrrolidone), poly(acrylic acid), poly(acrylic acid)-sodium salt, poly(acrylamide), poly(N-isopropyl acrylamide), poly(hydroxyethyl acrylate), poly(acrylic acid), poly(sodium styrene sulfonate), poly(2-acrylamido-2-methyl-1-propanesulfonic acid), polysaccharides, and cellulose derivatives. Preferably the porous layer has a porosity of 10-80%. In one embodiment, the thickness of the porous layer ranges from 0.01 µm to about 1,000 µm. Pore sizes included in the porous layer may range from 2 nm to about 100 µm.

According to another aspect there is disclosed a substrate comprising a porous polymeric material having a porosity from 10-80%, wherein reactive groups are chemically bound to the pore surfaces and are adapted in use to interact, e.g. by binding chemically, with a reactive species, e.g., deprotected monomeric building blocks or polymeric chains. In one embodiment the reactive group is a carboxylic acid group. The carboxylic acid group is free to bind, for example, an unprotected amine group of a peptide or polypeptide.

In an embodiment, the porous layer is in contact with a support layer. The support layer comprises, for example, metal, plastic, silicon, silicon oxide, or silicon nitride. In another embodiment, the porous layer may be in contact with a patterned surface, such as on top of pillar substrates described below.

### Pillar substrates

In some aspects, a substrate can include a planar layer comprising a metal and having an upper surface and a lower surface; and a plurality of pillars operatively coupled to the layer in positionally-defined locations, wherein each pillar has a planar surface extended from the layer, wherein the distance between the surface of each pillar and the upper surface of the layer is between about 1,000-5,000 angstroms, and wherein the plurality of pillars are present at a density of greater than about 10,000/ cm².

In some aspects, the distance between the surface of each pillar and the upper surface of the later can be between about less than 1,000, 2,000, 3,000, 3,500, 4,500, 5,000, or greater than 5,000 angstroms (or any integer in between).

In some aspects, the surface of each pillar is parallel to the upper surface of the layer. In some aspects, the surface of each pillar is substantially parallel to the upper surface of the layer.

In some aspects, the plurality of pillars are present at a density of greater than 500, 1,000, 2,000, 3,000, 4,000, 5,000, 6,000, 7,000, 8,000, 9,000, 10,000, 11,000, or 12,000/cm² (or any integer in between). In some aspects, the plurality of pillars are present at a density of greater than 10,000/cm². In some aspects, the plurality of pillars are present at a density of about 10,000/cm² to about 2.5 million/cm² (or any integer in between). In some aspects, the plurality of pillars are present at a density of greater than 2.5 million/cm².

In some aspects, the surface area of each pillar surface is at least 1 µm². In some aspects, the surface area of each pillar surface can be at least 0.1, 0.5, 12, 3, 4, 5, 6, 7, 8, 9, 10, 15, 20, 25, 30, 35, 40, 45, or 50 µm² (or any integer in between). In some aspects, the surface area of each pillar surface has a total area of less than 10,000 µm². In some aspects, the surface area of each pillar surface has a total area of less than 500, 1,000, 2,000, 3,000, 4,000, 5,000, 6,000, 7,000, 8,000, 9,000, 10,000, 11,000, or 12,000 µm² (or any integer in between).

In some aspects, the distance between the surface of each pillar and the lower surface of the layer is 2,000-7,000 angstroms. In some aspects, the distance between the surface of each pillar and the lower surface of the layer is about less than 500, 1,000, 2,000, 3,000, 4,000, 5,000, 6,000, 7,000, 8,000, 9,000, 10,000, 11,000, 12,000, or greater than 12,000 angstroms (or any integer in between). In some aspects, the distance between the surface of each pillar and the lower surface of the layer is 7,000, 3,000, 4,000, 5,000, 6,000, or 7,000 angstroms (or any integer in between).

In some aspects, the layer is 1,000-2,000 angstroms thick. In some aspects, the layer is about less than 500, 1,000, 2,000, 3,000, 4,000, 5,000, 6,000, 7,000, 8,000, 9,000, 10,000, 11,000, 12,000, or greater than 12,000 angstroms thick (or any integer in between).

In some aspects, the center of each pillar is at least 2,000 angstroms from the center of any other pillar. In some aspects, the center of each pillar is at least about 500, 1,000, 2,000, 3,000, or 4,000 angstroms (or any integer in between) from the center of any other pillar. In some aspects, the center of each pillar is at least about 2 µm to 200 µm from the center of any other pillar.

In some aspects, the metal is chromium. In some aspects, the metal is chromium, titanium, aluminum, tungsten, gold, silver, tin, lead, thallium, indium, or a combination thereof. In some aspects, the layer is at least 98.5-99% metal. In some aspects, the layer is 100% metal. In some aspects, the layer is at least about greater than 90, 91, 92, 93, 94, 95, 96, 97, 98, 98.5, or 99% metal. In some aspects, the layer is a homogenous layer of metal.

In some aspects, at least one or each pillar comprises silicon. In some aspects, at least one or each pillar comprises silicon dioxide or silicon nitride. In some aspects, at least one or each pillar is at least 90, 91, 92, 93, 94, 95, 96, 97, 98, 98.5, or 99% silicon dioxide.

In some aspects, a substrate can include a linker molecule having a free amino terminus attached to the surface of each pillar. In some aspects, a substrate can include a linker molecule having a free amino terminus attached to the surface of at least one pillar. In some aspects, a substrate can include a linker molecule having a protecting group attached to the surface of each pillar. In some aspects, a substrate can include a linker molecule having a protecting group attached to the surface of at least one pillar. In some aspects, a substrate can include a coupling molecule attached to the surface of at least one pillar. In some aspects, a substrate can include a coupling molecule attached to the surface of each pillar. In some aspects, a substrate can include a polymer in contact with the surface of at least one of the pillars. In some aspects, a substrate can include a polymer in contact with the surface of each pillar. In some aspects, a substrate can include a gelatinous form of a polymer in contact with the surface of at least one of the pillars. In some aspects, a substrate can include a solid form of a polymer in contact with the surface of at least one of the pillars.

In some aspects, the surface of at least one of the pillars of the substrate is derivatized. In some aspects, a substrate can include a polymer chain attached to the surface of at least one of the pillars. In some aspects, the polymer chain comprises a peptide chain. In some aspects, the attachment to the surface of the at least one pillar is via a covalent bond.

In some aspects, the surface of each pillar is square or rectangular in shape. In some aspects, the substrate can be coupled to a silicon dioxide layer. The silicon dioxide layer can be about 0.5 µm to 3 µm thick. In some aspects, the substrate can be coupled to a wafer, *e.g.,* a silicon wafer. The silicon dioxide layer can be about 700 µm to 750 µm thick.

### Arrays

Also disclosed herein are arrays. In some aspects, an array can be a three-dimensional array, *e.g.,* a porous array comprising features attached to the surface of the porous array. The surface of a porous array includes external surfaces and surfaces defining pore volume within the porous array. In some aspects, a three-dimensional array can include features attached to a surface at positionally-defined locations, said features each comprising: a collection of peptide chains of determinable sequence and intended length. In one embodiment, the fraction of polypeptides within said array is characterized by an average polypeptide conjugation efficiency for each coupling step of greater than 98%.

In some aspects, the average polypeptide conjugation efficiency is at least 98.5%. In some aspects, the average polypeptide conjugation efficiency is at least 99%. In some aspects, the average polypeptide conjugation efficiency for each coupling step is at least 90, 91, 92, 93, 94, 95, 96, 97, 98, 98.5, 98.6,98.7, 98.8, 98.9, 99.0, 99.1, 99.2, 99.3, 99.4, 99.5, 99.6, 99.7, 99.8, 99.9, or 100%.

In some aspects, an array can include at least 2, 10, 100, or 1,000 different polypeptide chains attached to the surface. In some aspects, an array can include at least 10,000 different polypeptide chains attached to the surface. In some aspects, an array can include at least 100, 500, 1000, 2000, 3000, 4000, 5000, 6000, 7000, 8000, 9000, 10,000, or greater than 10,000 different polypeptide chains attached to the surface (or any integer in between).

In some aspects, each of the positionally-defined locations is at a different, known location that is physically separated from each of the other positionally-defined locations. In some aspects, each of the positionally-defined locations is a positionally-distinguishable location. In some aspects, each determinable sequence is a known sequence. In some aspects, each determinable sequence is a distinct sequence.

In some aspects, the features are covalently attached to the surface. In some aspects, said peptide chains are attached to the surface through a linker molecule or a coupling molecule.

In some aspects, the features comprise a plurality of distinct, nested, overlapping peptide chains comprising subsequences derived from a source protein having a known sequence. In some aspects, each peptide chain in the plurality is substantially the same length. In some aspects, each peptide chain in the plurality is the same length. In some aspects, each peptide chain in the plurality is at least 5 amino acids in length. In some aspects, each peptide chain in the plurality is at least 5, 10, 15, 20, 25, 30, 35, 40, 45, 50, 55, or 60 amino acids in length. In some aspects, each peptide chain in the plurality is less than 5, at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, or greater than 60 amino acids in length. In some aspects, at least one peptide chain in the plurality is at least 5 amino acids in length. In some aspects, at least one peptide chain in the plurality is at least 5, 10, 15, 20, 25, 30, 35, 40, 45, 50, 55, or 60 amino acids in length. In some aspects, at least one peptide chain in the plurality is less than 5, at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, or greater than 60 amino acids in length. In some aspects, each polypeptide in a feature is substantially the same length. In some aspects, each polypeptide in a feature is the same length. In some aspects, the features comprise a plurality of peptide chains each having a random, determinable sequence of amino acids.

### Methods

### Methods of Manufacturing Substrates

Also disclosed herein are methods for making substrates. In some aspects, a method of producing a substrate can include coupling a porous layer to a support layer. The support layer may comprise any metal or plastic or silicon or silicon oxide or silicon nitride. In one embodiment, the substrate comprises multiple carboxylic acid substrates attached to the substrate for binding peptides during peptide synthesis and protein coupling. In some aspects, a method of producing a substrate can include coupling a porous layer to a plurality of pillars, wherein the porous layer comprises functional groups for attachment of a compound to the substrate, wherein the plurality of pillars are coupled to a planar layer in positionally-defined locations, wherein each pillar has a planar surface extended from the planar layer, wherein the distance between the surface of each pillar and the upper surface of the planar layer is between about 1,000-5,000 angstroms, and wherein the plurality of pillars are present at a density of greater than about 10,000/ cm².

In some aspects, the surface of each pillar is parallel to the upper surface of the planar layer. In some aspects, the surface of each pillar is substantially parallel to the upper surface of the planar layer.

In some aspects, a method of preparing a substrate surface can include obtaining a surface comprising silicon dioxide and contacted with a photoactive coupling formulation comprising a photoactive compound, a coupling molecule, a coupling reagent, a polymer, and a solvent; and applying ultraviolet light to positionally-defined locations located on the top of the surface and in contact with the photoactive formulation, wherein the surface area of each positionally-defined location on the surface has a total area of less than about 10,000/ µm². In some aspects, the method can include removing the photoactive formulation located external to the positionally-defined locations. In some aspects, the method can include reducing the thickness of the top of the surface located external to the positionally-defined locations. In some aspects, the method can include depositing a metal layer on the top of the surface with reduced thickness. In some aspects, the method can include removing the photoactive formulation in contact with the positionally-defined locations located on the top of the surface.

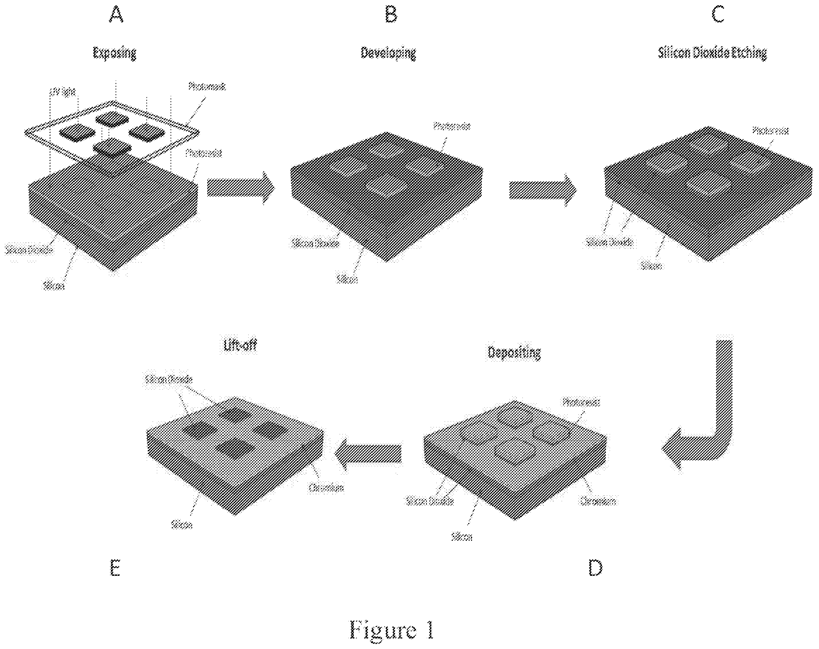

In one aspect, **Figs. 1A** - **1E** presents a process for producing a substrate.

Referring to **Fig. 1A****,** the first step in the preparation of a substrate is priming a starting wafer in order to promote good adhesion between a photoactive formulation (e.g., a photoresist) and a surface. Wafer cleaning can also be performed, which can include steps such as oxidation, oxide strip, and an ionic clean. Typically deionized (DI) water rinse is used to remove contaminants on the wafer surface. In wafer fabrication, silane deposition is generally needed to promote the chemical adhesion of an organic compound (photoresist) to a non-organic substrate (wafer). The silane acts as a sort of "bridge," with properties that will bond to both the photoresist and wafer surface. Typically, hexamethyldisilizane (HMDS) is used. HMDS is an organosilicon compound that is generally applied on heated substrates in gaseous phase in a spray module or in liquid phase through puddle and spin in a developer module followed by a bake step. In a puddle and spin method, HMDS is puddled onto the wafer for a specified time and then spun and baked at typical temperatures of 110-130°C for 1-2 mins. In a spray module, vapors of HMDS are applied onto a heated wafer substrate at 200-220 °C for 30s-50s.

Referring to **Fig. 1A****,** after wafer priming, the wafers can be coated with a deep ultra violet (DUV) photoresist in a photoresist coater module. DUV resists are typically polyhydroxystyrene-based polymers with a photoacid generator providing the solubility change. They can also comprise an optional photosensitizer. The matrix in the polymer consists of a protecting group for e.g., tboc attached to its end group.

The DUV resist is spin coated on the wafers in a photoresist coat module. This comprises a vacuum chuck held inside a cup. The wafers are mechanically placed on the chuck by, e.g., a robotic arm and then are spun at required speeds specified by the manufacturer to obtain the optimum thickness.

Referring to **Fig. 1A****,** the wafers are pre-heated in a pre-heat module. The pre-heat module typically includes a hot plate that can be set to required temperatures for the corresponding DUV resist as specified by the manufacturer. The heating can also be done in a microwave for a batch of wafers.

Referring to **Fig. 1A****,** the wafers are now exposed in a deep ultra violet radiation exposure tool through patterned photo masks.

Referring to **Fig. 1A****,** the wafers are now heated in a post exposure bake module. This post exposure leads to chemical amplification. The resist manufacturers provide the typical post exposure bake temperature and time for their corresponding product. When a wafer coated with a DUV photoresist is exposed to 248nm light source through a reticle, an initial photoacid or photobase is generated. The photoresist is baked to promote diffusion of the photoacid or photobase. The exposed portion of the resist becomes soluble to the developer thereby enabling patterning of 0.25 micron dimensions. A post exposure bake module comprises a hot plate set to the required temperatures as specified by the manufacturer. It can consist of three vacuum pins on which the wafers are placed by, e.g., a robotic arm. In other embodiments, the resist process does not use chemical amplification.

Referring to **Fig. 1B****,** the wafers are now developed in a developer module. A developer module typically consists of a vacuum chuck that can hold wafers and pressurized nozzles that can dispense the developer solution on to the wafers. The dispense mode can be a puddle and spin mode or a spin and rinse mode. Puddle and spin mode means the wafers remain stationery on the chuck for about 30sec to 1 minute when the developer solution is dispensed. This puddles the developer solution on top of the wafer. After a minute, it is spun away. In a spin and rinse mode, the developer solution is dispensed while the wafers are being spun.

Referring to **Fig. 1C****,** the oxide is now etched away in those regions that are developed by means of a wet etch or a dry etch process. Etching is a process by which material is removed from the silicon substrate or from thin films on the substrate surface. When a mask layer is used to protect specific regions of the wafer surface, the goal of etching is to precisely remove the material that is not covered by the mask. Normally, etching is classified into two types: dry etching and wet etching. Wet etching uses liquid chemicals, primarily acids to etch material, whereas dry etching uses gases in an excited state to etch material. These methods are well known to skilled artisans. These processes can be controlled to achieve an etch depth of, e.g., 1000A to 2000A.

Referring to **Fig. 1D**, a metal is deposited on the wafers. This metal is typically chromium, titanium, or aluminum. In some embodiments the metals are deposited by a process called sputter deposition. Sputter deposition is a physical vapor deposition (PVD) method of depositing thin films by sputtering, that is ejecting, material from a "target," that is a source, which then deposits onto the wafers. The thickness of metal deposition is ensured to be at least 500A on top of the substrate, if desired.

Referring to **Fig. 1E**, the photoresist in between the metal layer and the oxide can be lifted off by using the process diagrammed. In some aspects, the process includes lifting off the resist when the wafer has a metal layer without affecting the metal layer that previously has been deposited onto the silicon dioxide. This process results in lift off of the photoresist and metal deposited on the top surface of the substrate pillars, resulting in a silicon dioxide pillar rising above a metal-coated base that separates adjacent pillars. The wafers are submerged in an oxidizer solution overnight and then dipped in a Piranha solution for typically 1 hr. Piranha solution is a 1:1 mixture of sulfuric acid and hydrogen peroxide. This can be used to clean all the organic residues off the substrates. Since the mixture is a strong oxidizer, it will remove most of the organic matter, and it will also hydroxylate most surfaces (add OH groups), making them hydrophilic. This process can also include an additional step of plasma ashing.

### Surface Derivatization

Substrates can be surface derivatized in a semiconductor module as explained in U.S. Pat. App. Publication No. 2010/0240555 A1. A typical substrate used in the present invention has pillars of oxide ready to be surface derivatized. Surface derivatization is a method wherein an amino silane group is added to the substrate so that free amino groups are available for coupling the biomolecules. In some aspects, the first molecule to be attached to the surface derivatized substrate is a tboc protected Glycine. This coupling procedure is similar to a standard Merrifield solid phase peptide synthesis procedure which is generally known to one skilled in this art.

### Methods of Manufacturing Arrays

Also disclosed herein are methods for manufacturing arrays. In some aspects, the arrays disclosed herein can be synthesized in situ on a surface, *e.g.,* a substrate disclosed herein. In some instances, the arrays are made using photolithography. For example, the substrate is contacted with a photoactive conjugation solution. A photoactive compound in the photoactive conjugation solution binds to attachment groups (e.g., carboxylic acid or amine groups) attached to the surface of the array. Masks can be used to control radiation or light exposure to specific locations on a surface. In the exposed locations, the conjugation compounds are activated, resulting in one or more newly reactive moieties on the conjugation compound. The desired biomolecule or polypeptide is then coupled to the conjugation compound. The process can be repeated to synthesize a large number of features in specific or positionally-defined locations on a surface (see, for example, U.S. Pat. No. 5,143,854 to Pirrung et al., U.S. Patent Application Publication Nos. 2007/0154946 (filed on Dec. 29, 2005), 2007/0122841 (filed on Nov. 30, 2005), 2007/0122842 (filed on Mar. 30, 2006), 2008/0108149 (filed on Oct. 23, 2006), and 2010/0093554 (filed on June 2, 2008)).

In some aspects, a method of producing a two-dimensional array of features, can include obtaining a substrate comprising a planar layer comprising a metal and having an upper surface and a lower surface; and a plurality of pillars operatively coupled to the layer in positionally-defined locations, wherein each pillar has a planar surface extended from the layer, wherein the distance between the surface of each pillar and the upper surface of the layer is between about 1,000-5,000 angstroms, and wherein the plurality of pillars are present at a density of greater than about 10,000/ cm²; and coupling through a series of coupling reactions the features to the plurality of pillars, said features each comprising a known biomolecule or polypeptide. In some embodiments, the average coupling efficiency of conjugation of a biomolecule to conjugation compound attached to the array is at least about 98%. In some embodiments, the average coupling efficiency of conjugation of a biomolecule to conjugation compound attached to the array exceeds 98%. In some aspects, the features are coupled to the pillars using a conjugation solution, comprising a conjugation compound, a polymer, and a solvent. The conjugation solution is added to the array and the conjugation compound is attached to the array. The conjugation solution is removed from the array by, e.g., washing with water. A solution comprising the features (e.g., biomolecules) is added to the array. The array is selectively exposed to electromagnetic radiation through, e.g., a photomask or reticle. Sites exposed to electromagnetic radiation have attached activated conjugation compounds that bind to the features in solution. This process may be repeated so that sites that are unbound to a feature are activated to bind to different features from a new solution of features. In one aspect, an array comprising at least two distinct features is produced. In one aspect, an array comprising at least ten distinct features is produced. In one aspect, an array comprising at least 100 distinct features is produced. In one aspect, an array comprising at least 1,000 distinct features is produced. In one aspect, an array comprising at least 10,000 distinct features is produced. In one aspect, an array comprising at least 2, 5, 10, 20, 50, 100, 200, 500, 1,000, 2,000, 5,000, 10,000, 20,000, 50,000, 100,000, 200,000, 500,000, or 1,000,000 distinct features is produced.

In some aspects, a method of preparing a surface for attachment of features (e.g., biomolecules), can include obtaining a surface and attaching a linker molecule to the surface using a linker formulation, comprising a solvent, a polymer, a linker molecule, and a coupling reagent. In some aspects, the linker molecule comprises a protecting group.

In some aspects, a method of attaching a coupling reagent to a substrate, can include obtaining a substrate comprising a planar layer comprising a metal and having an upper surface and a lower surface; and a plurality of pillars operatively coupled to the layer in positionally-defined locations, wherein each pillar has a planar surface extended from the layer, wherein the distance between the surface of each pillar and the upper surface of the layer is between 1,000-5,000 angstroms, wherein a linker molecule is attached to the surface of each pillar, and wherein the plurality of pillars are present at a density of greater than 10,000/ cm²; and attaching the conjugation compound to one or more linker molecules. In some aspects, the conjugation compound is attached to the one or more linker molecules using a conjugation solutions, comprising: a solvent, a polymer, and a conjugation compound. In some aspects, the conjugation compound is attached to the one or more linker molecules using a conjugation solution disclosed herein. In some aspects, at least one the linker molecule is a deprotected linker molecule. In some aspects, the conjugation compound is an NHS ester of a photoactive compound. In some aspects, the conjugation compound is a carbodiimide ester of a photoactive compound. In some aspects, the conjugation compound is an amine of a photoactive compound. In some aspects, the conjugation compound comprises a protecting molecule. In some aspects, the conjugation solution is stripped away using water. In some aspects, the conjugation compounds are activated with UV radiation or light at site-specific locations (e.g., selected pillars). In some aspects, a feature is added to the activated conjugation compound and bound to the substrate. In some aspects, the surface of each pillar is parallel to the upper surface of the layer. In some aspects, the surface of each pillar is substantially parallel to the upper surface of the layer.

In some aspects, a method of producing a three-dimensional (e.g., porous) array of features, can include obtaining a porous layer attached to a surface, wherein the surface comprises attachment groups; and attaching the conjugation groups to the attachment groups. The conjugation groups are then site-selectively activated via electromagnetic radiation through a photomask or reticle, and the activated conjugation groups binds to a desired polypeptide added to the surface of said array. The fraction of polypeptides binding to said conjugated groups is characterized by an average conjugation efficiency of at least about 98%. In some aspects, the features are attached to the surface using a photoactive conjugation solution, comprising a photoactive conjugation compound, a polymer, and a solvent, followed by addition of the polypeptide and activation of the attached conjugation compound.

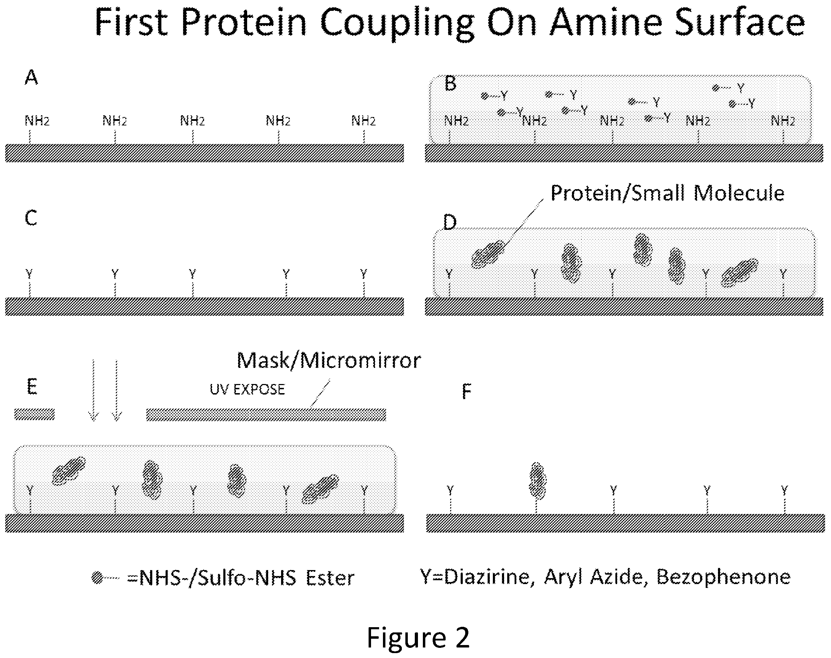

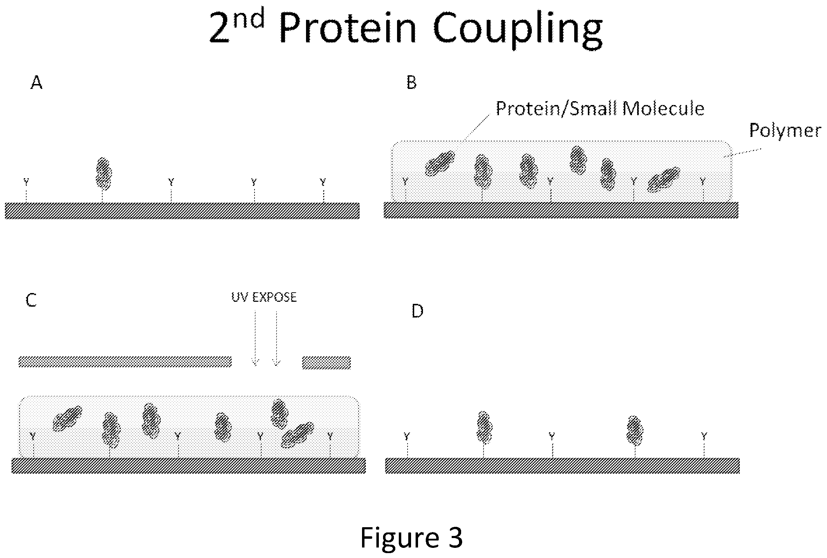

In one embodiment, **Figures 2** **and** **3** describe a process of manufacturing an array. Referring to **Figure 2A****,** a surface comprising attached amine groups is provided. The surface is contacted with a conjugation solution comprising a photoactive conjugation compound, a polymer, and a solvent **(****Figure 2B**). The photoactive conjugation compound comprises an activated carboxylic acid group for binding to the amine group on the surface of the array, allowing the conjugation compound to bind to the amine group on the surface of the array **(****Figure 2C**). The conjugation solution is then stripped from the array. The surface is contacted with a biomolecule coupling solution comprising a biomolecule, a polymer, and a solvent **(****Figure 2D****).** The surface is exposed to ultraviolet light in a deep ultra violet scanner tool according to a pattern defined by a photomask, wherein the locations exposed to ultraviolet light undergoes photoactivation of the photoactive conjugation compound **(****Figure 2E**). The expose energy can be from 1mJ/cm² to 100mJ/cm² in order to activate the photoactive conjugation compound. In one aspect activation of the photoactive conjugation compound generates a carbene group that is highly reactive to any X-H bond on the biomolecule.

The surface is post baked upon exposure in a post exposure bake module. The post bake temperature can vary between 75°C to 115°C, depending on the thickness of the surface, for at least 60 sec and not usually exceeding 120 sec. The photoactivated conjugation compound is coupled to the biomolecule, resulting in coupling of the biomolecule to the surface of the array in a site-specific manner **(****Figure 2F**). This surface may be a porous surface.

This entire cycle can be repeated as desired with different coupling molecules each time to obtain a desired sequence **(****Figure 3A-D**).

Optionally, a cap film solution coat is applied on the surface to prevent the unreacted amine groups on the substrate from reacting with a biomolecule. The cap film coat solution can be prepared as follows: a solvent, a polymer, and a coupling molecule.

This process is done in a capping spin module. A capping spin module can include one nozzle that can be made to dispense the cap film coat solution onto the substrate. This solution can be dispensed through pressurizing the cylinder that stores the cap film coat solution or through a pump that precisely dispenses the required amount. In some aspects, a pump is used to dispense around 5-8 cc of the cap coat solution onto the substrate. The substrate is spun on a vacuum chuck for 15-30 s and the coupling formulation is dispensed. The spin speed can be set to 2000 to 2500 rpm.

The substrates with the capping solution are baked in a cap bake module. A capping bake module is a hot plate set up specifically to receive wafers just after the capping film coat is applied. In some aspects, provided herein is a method of baking the spin coated capping coat solution in a hot plate to accelerate the capping reaction significantly. Hot plate baking generally reduces the capping time for amino acids to less than two minutes.

The byproducts of the capping reaction are stripped in a stripper module. A stripper module can include several nozzles, typically up to 10, set up to dispense organic solvents such as acetone, iso propyl alcohol, N methyl pyrrolidone, Di methyl formamide, DI water, etc. In some aspects, the nozzles can be designated for acetone followed by iso propyl alcohol to be dispensed onto the spinning wafer. The spin speed is set to be 2000 to 2500 rpm for around 20 s.

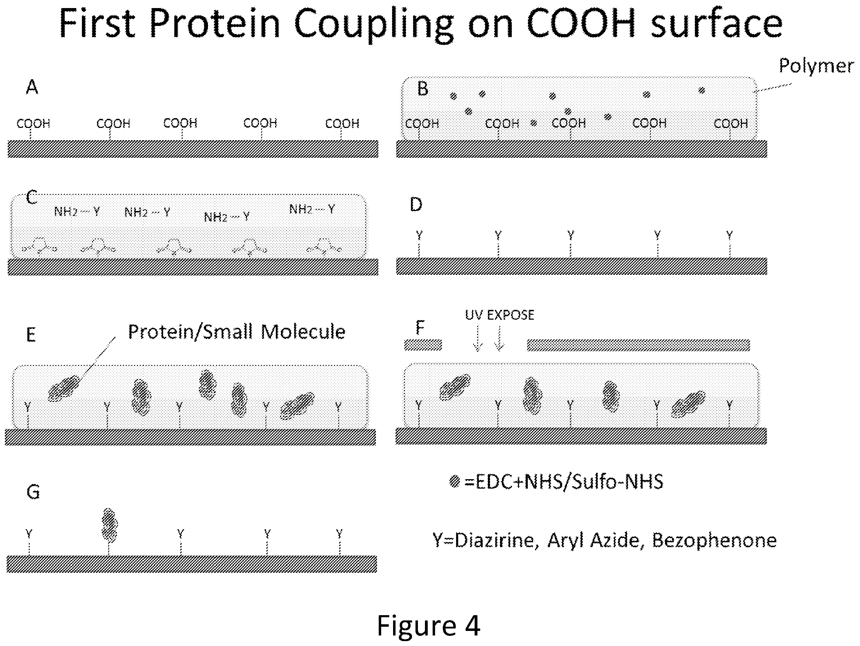

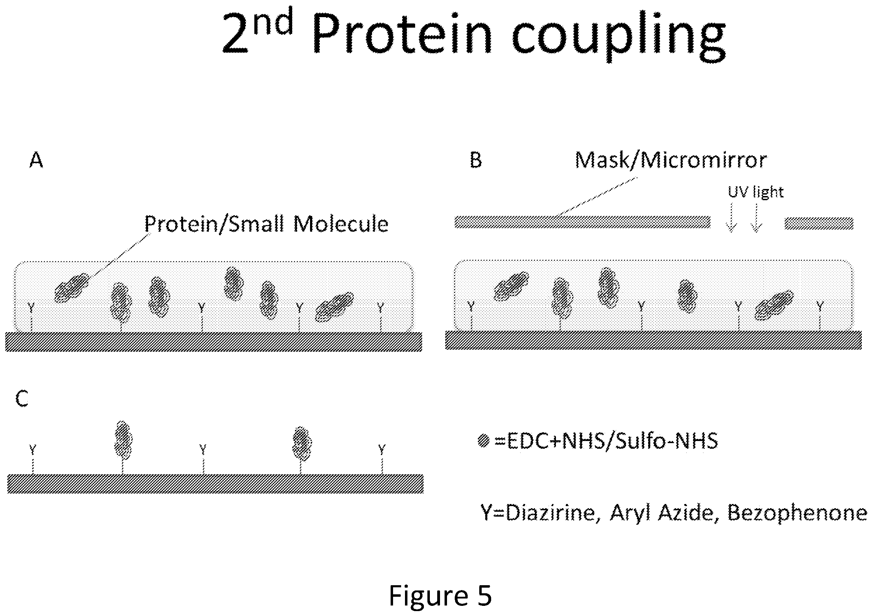

In one embodiment, **Figure 4** **and** **5** describes a process of manufacturing an array. Referring to **Figure 4A****,** a surface comprising attached carboxylic acid groups is provided. The carboxylic acid groups are activated by addition of a carboxylic group activating solution **(****Figure 4B**). In one embodiment, the carboxylic acid group activating solution comprises a carbodiimide or a succinimide. In one embodiment, the carboxylic acid group activating solution comprises 1-ethyl-3-(3-dimethylaminopropyl) carbodiimide, N,N'-diisopropylcarbodiimide, (Benzotriazol-1-yloxy)tripyrrolidinophosphonium hexafluorophosphate, bromo(tripyrrolidin-1-yl)phosphonium hexafluorophosphate, O-(7-azabenzotriazol-1-yl)-N,N,N',N'-tetramethyluronium hexafluorophosphate, O-Benzotriazole-N,N,N',N'-tetramethyl-uronium-hexafluoro-phosphate, or O-(Benzotriazol-1-yl)-N,N,N',N'-tetramethyluronium tetrafluoroborate. The surface is contacted with a conjugation solution comprising a photoactive conjugation compound, a polymer, and a solvent **(****Figure 4C**). The photoactive conjugation compound comprises an amine group for binding to the activated carboxylic acid group on the surface of the array. After binding to the activated carboxylic acid, excess photoactive conjugation solution is washed away **(****Figure 4D**). The surface is then contacted with a biomolecule coupling solution comprising a biomolecule, a polymer, and a solvent **(****Figure 4E**). The surface is exposed to ultraviolet light in a deep ultra violet scanner tool according to a pattern defined by a photomask, wherein the locations exposed to ultraviolet light undergo photoactivation of the photoactive conjugation compound **(****Figure 4F**). The expose energy can be from 1mJ/cm² to 100mJ/cm² in order to activate the photoactive conjugation compound. In one aspect activation of the photoactive conjugation compound generates a carbene group that is highly reactive to any X-H bond on the biomolecule.

The surface is post baked upon exposure in a post exposure bake module. The post bake temperature can vary between 75°C to 115°C, depending on the thickness of the surface, for at least 60 sec and not usually exceeding 120 sec. The photoactivated conjugation compound is coupled to the biomolecule, resulting in coupling of the biomolecule to the surface of the array in a site-specific manner **(****Figure 4G**). This surface may be a porous surface.

This entire cycle can be repeated as desired with different coupling molecules each time to obtain a desired sequence **(****Figure 5A-C**).

Optionally, a cap film solution coat is applied on the surface to prevent the unreacted carboxylic acids on the substrate from reacting with a biomolecule. The cap film coat solution can be prepared as follows: a solvent, a polymer, and a coupling molecule. The solvent that can be used can be an organic solvent like N methyl pyrrolidone, di methyl formamide, or combinations thereof. The capping molecule is typically acetic anhydride and the polymer can be Poly vinyl pyrrolidone, polyvinyl alcohol, polymethyl methacrylate, poly (methyl iso propenyl) ketone, or poly (2 methyl pentene 1 sulfone). In some embodiments, the capping molecule is ethanolamine.

This process is done in a capping spin module. A capping spin module can include one nozzle that can be made to dispense the cap film coat solution onto the substrate. This solution can be dispensed through pressurizing the cylinder that stores the cap film coat solution or through a pump that precisely dispenses the required amount. In some aspects, a pump is used to dispense around 5-8 cc of the cap coat solution onto the substrate. The substrate is spun on a vacuum chuck for 15-30 s and the coupling formulation is dispensed. The spin speed can be set to 2000 to 2500 rpm.

The substrates with the capping solution are baked in a cap bake module. A capping bake module is a hot plate set up specifically to receive wafers just after the capping film coat is applied. In some aspects, provided herein is a method of baking the spin coated capping coat solution in a hot plate to accelerate the capping reaction significantly. Hot plate baking generally reduces the capping time for amino acids to less than two minutes.

The byproducts of the capping reaction are stripped in a stripper module. A stripper module can include several nozzles, typically up to 10, set up to dispense organic solvents such as acetone, iso propyl alcohol, N methyl pyrrolidone, Di methyl formamide, DI water, etc. In some aspects, the nozzles can be designated for acetone followed by iso propyl alcohol to be dispensed onto the spinning wafer. The spin speed is set to be 2000 to 2500 rpm for around 20 s.

### Methods of Use

Also disclosed herein are methods of using substrates, formulations, and/or arrays. Uses of the arrays disclosed herein can include research applications, therapeutic purposes, medical diagnostics, and/or stratifying one or more patients.

Any of the arrays described herein can be used as a research tool or in a research application. In one aspect, arrays can be used for high throughput screening assays. For example, enzyme substrates (*i.e.,* polypeptides on a peptide array described herein) can be tested by subjecting the array to an enzyme and identifying the presence or absence of enzyme substrate(s) on the array, e.g., by detecting at least one change among the features of the array.

Arrays can also be used in screening assays for ligand binding, to determine substrate specificity, or for the identification of polypeptides that inhibit or activate proteins. Labeling techniques, protease assays, as well as binding assays useful for carrying out these methodologies are generally well-known to one of skill in the art.

In some aspects, an array is used for high throughput screening of one or more genetic factors. Proteins associated with a gene can be a potential antigen and antibodies against these gene related proteins can be used to estimate the relation between gene and a disease.

In another example, an array can be used to identify one or more biomarkers. Biomarkers can be used for the diagnosis, prognosis, treatment, and management of diseases. Biomarkers may be expressed, or absent, or at a different level in an individual, depending on the disease condition, stage of the disease, and response to disease treatment. Biomarkers can be, *e.g.,* DNA, RNA, proteins *(e.g.,* enzymes such as kinases), sugars, salts, fats, lipids, or ions.

Arrays can also be used for therapeutic purposes, *e.g*., identifying one or more bioactive agents. A method for identifying a bioactive agent can comprise applying a plurality of test compounds to an array and identifying at least one test compound as a bioactive agent. The test compounds can be small molecules, aptamers, oligonucleotides, chemicals, natural extracts, peptides, proteins, fragment of antibodies, antibody like molecules or antibodies. The bioactive agent can be a therapeutic agent or modifier of therapeutic targets. Therapeutic targets can include phosphatases, proteases, ligases, signal transduction molecules, transcription factors, protein transporters, protein sorters, cell surface receptors, secreted factors, and cytoskeleton proteins.

In another aspect, an array can be used to identify drug candidates for therapeutic use. For example, when one or more epitopes for specific antibodies are determined by an assay (*e.g.,* a binding assay such as an ELISA), the epitopes can be used to develop a drug (*e.g.,* a monoclonal neutralizing antibody) to target antibodies in disease.

In one aspect, also provided are arrays for use in medical diagnostics. An array can be used to determine a response to administration of drugs or vaccines. For example, an individual's response to a vaccine can be determined by detecting the antibody level of the individual by using an array with peptides representing epitopes recognized by the antibodies produced by the induced immune response. Another diagnostic use is to test an individual for the presence of biomarkers, wherein samples are taken from a subject and the sample is tested for the presence of one or more biomarkers.

Arrays can also be used to stratify patient populations based upon the presence or absence of a biomarker that indicates the likelihood a subject will respond to a therapeutic treatment. The arrays can be used to identify known biomarkers to determine the appropriate treatment group. For example, a sample from a subject with a condition can be applied to an array. Binding to the array may indicate the presence of a biomarker for a condition. Previous studies may indicate that the biomarker is associated with a positive outcome following a treatment, whereas absence of the biomarker is associated with a negative or neutral outcome following a treatment. Because the patient has the biomarker, a health care professional may stratify the patient into a group that receives the treatment.

In some aspects, a method of detecting the presence or absence of a protein of interest (e.g., an antibody) in a sample can include obtaining an array disclosed herein and contacted with a sample suspected of comprising the protein of interest; and determining whether the protein of interest is present in the sample by detecting the presence or absence of binding to one or more features of the array. In some aspects, the protein of interest may be obtained from a bodily fluid, such as amniotic fluid, aqueous humour, vitreous humour, bile, blood serum, breast milk, cerebrospinal fluid, cerumen, chyle, endolymph, perilymph, feces, female ejaculate, gastric acid, gastric juice, lymph, mucus, peritoneal fluid, pleural fluid, pus, saliva, sebum, semen, sweat, synovial fluid, tears, vaginal secretion, vomit, or urine.

In some aspects, a method of identifying a vaccine candidate can include obtaining an array disclosed herein contacted with a sample derived from a subject previously administered the vaccine candidate, wherein the sample comprises a plurality of antibodies; and determining the binding specificity of the plurality of antibodies to one or more features of the array.

### EXAMPLES

Below are examples of specific aspects of the present disclosure. The examples are offered for illustrative purposes only. Efforts have been made to ensure accuracy with respect to numbers used (*e.g*., amounts, temperatures, etc.), but some experimental error and deviation should, of course, be allowed for.

The practice of the present invention will employ, unless otherwise indicated, conventional methods of protein chemistry, biochemistry, recombinant DNA techniques and pharmacology, within the skill of the art. Such techniques are explained fully in the literature. See, *e.g.,* T.E. Creighton, Proteins: Structures and Molecular Properties (W.H. Freeman and Company, 1993); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences, 18th Edition (Easton, Pennsylvania: Mack Publishing Company, 1990); Carey and Sundberg Advanced Organic Chemistry 3rd Ed. (Plenum Press) Vols A and B (1992).

### Example 1: Production of a pillar substrate.

This example describes construction of a substrate with surfaces on top of pillars. This process is visually outlined in **Figure 1****.** Silicon wafers with 2.4µm thermally grown oxide were obtained from University Wafers. These wafers were first primed with a primer in a spray module. Hexamethyl disilazane (HMDS) was obtained from Sigma Aldrich Inc. The wafers were then spun coat in a photoresist coat module with a commercially available deep Ultra violet photoresist, P5107 obtained from Rohm and Haas or AZ DX7260p 700 from AZ Electronic Materials, to obtain a thickness of 6000Å. The wafers were then baked in a hot plate at 120°C for 60 seconds.

Photomasks that have the patterned regions to create the features were used to image the array on to the substrate surface. The wafers were then exposed in a 248nm deep ultra violet radiation scanner tool, Nikon S203, with expose energy of 18mJ/cm2. The wafers were then post exposure baked at 110°C for 120 seconds in a hot plate and developed with commercially available NMD-3 developer, obtained from Tokyo Ohka Kogyo Co., Ltd., for 60 seconds.

After this the oxide was etched by using either a wet etch process or dry plasma etch process. Standard semiconductor etch techniques were used. Oxide etch depths were from 1000Å to 2000Å.

After etching, chromium was deposited to a thickness of 500Å to 1500Å by a physical deposition method. Standard etching and metal deposition techniques were employed.

After the chromium was deposited, the resist was lifted off with the following process: The wafer was left in Nanostrip obtained from Cyantek Inc. overnight and then dipped in Piranha solution for 90 mins. Piranha solution is a 50:50 mixture of sulfuric acid and hydrogen peroxide. Sulfuric acid and hydrogen peroxide were obtained from Sigma Aldrich Corp. Plasma ashing was performed to oxidize the remaining impurities. This process produced a substrate having pillars of silicon dioxide separated by metal.

Alternatively, the deposited chromium was also polished to a depth of 500Å to 1500 A, depending on the deposition. The polishing was performed to obtain pillars of silicon dioxide separated by metal. The separation of each pillar from center to center was 70,000Å. The surface area of top of each pillar was 3,500Å × 3,500Å.

### Example 2: Surface derivatization with an amine group.

The wafers from Example 1 were surface derivatized using the following method: Aminopropyl triethoxy silane (APTES) was obtained from Sigma Aldrich. Ethanol 200 proof was obtained from VWR. The wafers were first washed with ethanol for 5 minutes and then in 1% by weight APTES/Ethanol for 20-30 minutes to grow the silane layer. Then the wafers were cured in a 110°C nitrogen bake oven to grow a mono silane layer with a -NH₂ group to attach a linker molecule.

### Example 3: Surface derivatization with a carboxylic acid group.

Silicon wafers deposited with Nickel 1000A on a silicon substrate were obtained from University Wafers. Dextran Bio Xtra (MW40000) was obtained from Sigma Aldrich. Bis-Polyethylene glycol carboxy methyl ether was obtained from Sigma Aldrich. Poly vinyl pyrollidone 1000000 was obtained from Poly Sciences Inc. The above three polymers were dissolved in a solvent composition of 50% Ethyl lactate/ 50% water by weight in a ratio of 2:2:1 by weight along with 2% by weight photoacid generator dimethyl-2,4-dihydroxyphenylsulfonium triflate obtained from Oakwood Chemicals Inc. This solution was spin coated onto a silicon wafer deposited with deposited with Nickel 1000A on a silicon substrate.

The coated wafer was spun at 3000rpm to obtain a uniform coat of thickness 100nm. The wafer was then exposed in a deep UV scanner Nikon S 203 at 250mJ/cm² and then baked at 65°C for 90 seconds in a hot plate. The coating was then stripped off the wafer with acetone and isopropyl alcohol followed by a deionized water rinse. The substrate has a matrix of free COOH groups ready to be activated and coupled with a protein or an amino acid for peptide synthesis.

The above derivatization can be performed on the surface of the pillars from the pillar substrates of Example 1.

### Example 4: Production of a dextran-based porous substrate coated with carboxylic acid groups.

The 2-dimensional concentration of COOH groups along the layer may be increased on a porous substrate as compared to a planar substrate. Dextran was coupled onto a surface derivatized wafer. 1-ethyl-3-(3-dimethylaminopropyl) carbodiimide obtained from Pierce Scientific and N-Hydroxysuccinimide (NHS) obtained from Pierce Scientific were dissolved in deionized water in molar concentration of 0.2M and 0.1M respectively along with 10% by weight of Dextran. This coupling solution was spin coated to the wafer at a speed of 3000rpm and baked at 65°C for 90 seconds to complete coupling of dextran -COOH substrate. Crosslinking solution was added and crosslinked to provide a multidimensional COOH substrate.

### Example 5: Production of a PEG-based porous substrate coated with carboxylic acid groups.

Bis-Polyethylene glycol carboxy methyl ether was coupled onto a surface derivatized wafer. 1-ethyl-3-(3-dimethylaminopropyl) carbodiimide obtained from Pierce Scientific and N-Hydroxysuccinimide (NHS) obtained from Pierce Scientific were dissolved in deionized water in molar concentration of 0.2M and 0.1M respectively along with 10% by weight of polyethylene glycol (PEG). This coupling solution was spin coated to the wafer at a speed of 3000rpm and baked at 65°C for 90 seconds to complete coupling of PEG-COOH substrate. Crosslinking solution was added and crosslinked to provide a multidimensional COOH substrate.

### Example 6: Production of a hydroxyl group derivatized pillar surface on a substrate.

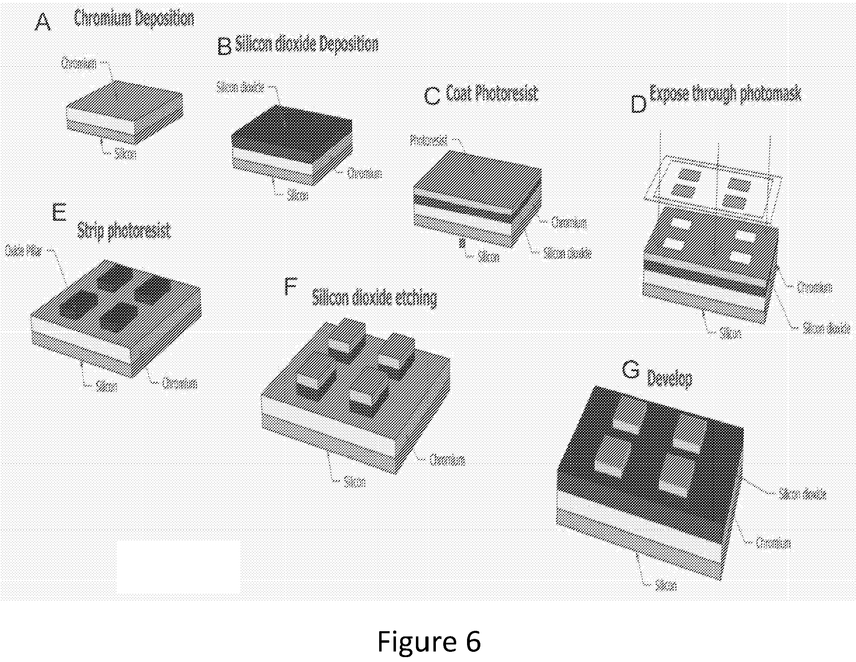

Silicon wafers were obtained from University Wafers. Referring to **Figure** 6A a metal was deposited on the wafers. This metal was selected from chromium, titanium, or aluminum. The metals were deposited by a process called sputter deposition. Sputter deposition is a physical vapor deposition (PVD) method of depositing thin films by sputtering, that is ejecting, material from a "target," that is a source, which then deposits onto the wafers. The thickness of metal deposition was ensured to be at least 500Å on top of the substrate.

Referring to **Figure 6B**, silicon dioxide was deposited on the wafers. The oxide was deposited by a process called sputter deposition. Sputter deposition is a physical chemical vapor deposition (PECVD) method of depositing thin films by sputtering, that is ejecting, material from a "target," that is a source, which then deposits onto the wafers. The thickness of oxide deposition was ensured to be at least 500Å on top of the substrate.

Referring to **Figure 6C**, the first step in the preparation of a substrate was priming a starting wafer in order to promote good adhesion between a photoactive formulation (e.g., a photoresist) and a surface. Wafer cleaning was also performed, which included oxidation, oxide strip, and an ionic clean. (DI) water rinse was used to remove contaminants on the wafer surface. In wafer fabrication, silane deposition was used to promote the chemical adhesion of an organic compound (photoresist) to a non-organic substrate (wafer). The silane acts as a sort of "bridge," with properties bind to both the photoresist and wafer surface. Typically, hexamethyldisilizane (HMDS) was used. HMDS is an organosilicon compound that was applied on heated substrates in gaseous phase in a spray module or in liquid phase through puddle and spin in a developer module. This was followed by a bake step. In a puddle and spin method, HMDS was puddled onto the wafer for a specified time and then was spun and baked at temperatures of 110°C-130°C for 1-2 mins. In a spray module, vapors of HMDS were applied onto a heated wafer substrate at 200°C-220°C for 30s-50s.

Referring **to** **Fig. 6C**, after wafer priming, the wafers were coated with a deep ultra violet (DUV) photoresist in a photoresist coater module. Our DUV resist comprised polyhydroxystyrene-based polymers with a photoacid generator providing the solubility change. The DUV resist further comprised a photosensitizer. The matrix in the polymer comprised a protecting group for e.g., tboc attached to the end group.

The DUV resist was spin coated on the wafers in a photoresist coat module. This module comprised a vacuum chuck held inside a cup. The wafers were mechanically placed on the chuck by a robotic arm and then were spun at required speeds specified by the manufacturer to obtain the optimum thickness.

Referring to **Fig. 6C****,** the wafers were pre-heated in a pre-heat module. The pre-heat module included a hot plate that can be set to required temperatures for the corresponding DUV resist as specified by the manufacturer. In cases for heating a batch of wafers, we used a microwave for heating.

Referring to **Fig. 6D****,** the wafers were exposed in a deep ultra violet radiation exposure tool through patterned photo masks.

Referring to **Fig. 6E****,** the wafers were heated in a post exposure bake module. This post exposure led to chemical amplification. The resist manufacturers provided the typical post exposure bake temperature and time for their corresponding product. When a wafer coated with a DUV photoresist was exposed to 248nm light source through a reticle, an initial photoacid or photobase was generated. The exposed portion of the resist became soluble to the developer thereby enabling patterning of 0.25 micron dimensions. A post exposure bake module comprised a hot plate set to the required temperatures as specified by the manufacturer. The module comprised three vacuum pins on which the wafers were placed by a robotic arm.

Referring to **Fig. 6E****,** the wafers were developed in a developer module. The developer module comprised a vacuum chuck that held wafers and pressurized nozzles that dispensed the developer solution on to the wafers. The dispense mode was either a puddle and spin mode or a spin and rinse mode. During the puddle and spin mode, the wafers remained stationery on the chuck for about 30 seconds to 1 minute when the developer solution was dispensed. This puddled the developer solution on top of the wafer. After one minute, the developer solution was spun away. During the spin and rinse mode, the developer solution was dispensed while the wafers were spun.

Referring to **Fig. 6F****,** the oxide was etched away in those regions that are developed by means of a wet etch or a dry etch process. Etching is a process by which material is removed from the silicon substrate or from thin films on the substrate surface. When a mask layer is used to protect specific regions of the wafer surface, the goal of etching is to precisely remove the material, which is not covered by the mask. Normally, etching is classified into two types: dry etching and wet etching. Wet etching uses liquid chemicals, primarily acids to etch material, whereas dry etching uses gases in an excited state to etch material. These processes were run to achieve an etch depth of, e.g., 500Å.

Referring to **Fig. 6G**, the wafers were submerged in an oxidizer solution overnight and then dipped in a Piranha solution for typically 1 hr. Piranha solution used was a 1:1 mixture of sulfuric acid and hydrogen peroxide. This solution was used to clean all the organic residues off the substrates. Since the mixture is a strong oxidizer, it removed most of the organic matter, and it hydroxylated most surfaces (i.e., add OH groups to the surface), making the surfaces hydrophilic. This process also included an additional step of plasma ashing.

### Example 7: Conjugation of an IL-6 and TNF alpha protein to an amine group derivatized surface.

The wafers are surface derivatized as explained in Example 2 to achieve an amine group on the substrate (**Figure 2A**). Photo conjugation groups such as carboxylic NHS esters of diazirine, aryl azide or benzophenone are obtained from Sigma Aldrich. 0.1mM of NHS-diazirine is dissolved in 1%PVP/water to create a conjugation solution. PVP (Polyvinyl pyrrollidone) was obtained from Polysciences. The conjugation solution is spin coated onto a wafer at 2000rpm for 30 secs and is left standing for 30 mins to complete coupling (**Figure 2B**). This process of coupling can also be done by heating in a bake oven or microwave to improve coupling efficiency and also reduce time. The wafers with conjugation solution were washed with tris buffered saline, obtained from VWR, to quench the unreacted NHS (**Figure 2C**). Capping solution is prepared as follows: 50% Acetic anhydride obtained from Spectrum chemicals and 50% N Methyl pyrrollidone, obtained from VWR, is mixed. The capping solution is coated on the wafer and baked for 90 seconds at 75°C to cap the unreacted amine. Now the wafer is washed with N-methyl pyrrollidone followed by DI water rinse and dry. Recombinant IL-6 was obtained from Life Tech. IL-6 coupling solution is prepared by dissolving 50ug/ml of IL-6 and 1% PVP in deionized water. This protein coupling solution is spin coat on wafer at 2000 rpm for 30 sec (**Figure 2D**). The wafer is now exposed using deep UV light at 248nm in a Nikon S203 Scanner with a reticle at 100mJ/cm² (**Figure 2E**). This can also be done with a digital micromirror or other maskless lithography based systems. During exposure the UV photolysis of diazirene forms carbene that is highly reactive with any X-H bonds in proteins like IL-6 to form a stable covalent bond. The protein coupling solution is then washed off the array to leave bound IL-6 at site-specific locations (**Figure 2F**). This process completes one protein conjugation.

The steps above are repeated for coupling TNF alpha to site-specific spots different from those coupled to IL-6 using a different reticle to expose a different spot **(****Figure 3A-3D**). These steps can be repeated several times to couple selected polypeptides to specific spots on an array.

To test binding of IL-6 and TNF alpha to the array, anti-TNF alpha and anti-IL-6 antibodies are added with a dilution of 1:1000 and mixed together in a PBST buffer. All antibodies and buffer solutions are obtained from Life Technologies. The assay was performed as follows: Chips were washed in PBST buffer thrice for 5 minutes. Next, the antibodies were added and incubated for 1 hour at 37°C in the dark. Next, the chips were washed with PBST buffer thrice for 5 minutes followed by deionized water thrice for 5 minutes. Finally, the chips were scanned in a fluorescent scanner.

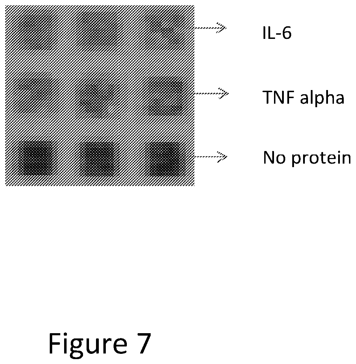

Fluorescence signal intensity for IL-6 is measured to be 45000 and fluorescence signal intensity for TNF alpha is measured to be 43500 compared to fluorescence signal intensity of no protein at 1500 (**Figure 7**). This result proves that coupling of two or more proteins can be achieved in an array.

Since the intermediate carbene formed is highly reactive with any X-H bond, this microarray based photoconjugation can be extended to cover small molecules and any chemical or bio molecule that comprises of X-H bond. In the case of benzophenone, photolysis at deep UV causes it to react with C-H bonds. Thus photoconjugation of proteins in a microarray format one at a time can be used to not only generate an array comprising antibodies and other macro biomolecules, but also can be used to develop an array comprising small molecules.

### Example 8: Conjugation of an IL-6 and TNF alpha protein to an carboxylic acid group derivatized surface..

Wafers surface derivatized as explained in Example 6 to achieve a COOH group on the substrate are provided (**Figure 4A**). The wafer is activated with EDC/NHS, obtained from Sigma Aldrich, for 10 minutes at room temperature (**Figure 4B**).

Photo conjugation groups such as amino diazirine, aryl azide or benzophenone are obtained from Life Tech. 0.1mM of amino diazirine is dissolved in 1%PVP/water. PVP (Polyvinyl pyrrollidone) was obtained from Polysciences. This conjugation solution is spin coated onto a wafer at 2000rpm for 30 seconds and is left standing for 30 minutes to complete coupling (**Figure 4C**). This process of coupling can also be done by heating in a bake oven or microwave to improve coupling efficiency and also reduced time. The wafers were washed with tris buffered saline, obtained from VWR **(****Figure 4D**). Capping solution is prepared as follows: 1M ethanolamine, obtained from Sigma Aldrich is dissolved in DI water and 1% PVP and spin coated onto the wafer. The coat was allowed to stand for 10 minutes at room temperature. Next, the wafer is washed with deionized water and dried. Recombinant IL-6 was obtained from Life Tech. IL-6 coupling solution is prepared by dissolving 50µg/ml of IL-6 and 1% PVP in deionized water. This protein coupling solution was spin coated on a wafer at 2000 rpm for 30 seconds **(****Figure 4E**). The wafer was then exposed to deep UV light at 248nm in a Nikon S203 Scanner with a reticle at 100mJ/cm² **(****Figure 4F**). This can also be done with a digital micromirror or other maskless lithography based systems as well as in a 365nm stepper/scanner. During exposure the UV photolysis of diazirene forms carbene that is highly reactive with any X-H bonds in proteins like IL-6 to form a stable covalent bond between IL-6 and the conjugation compound. Excess protein coupling solution was then washed off the wafer. This process completes on protein coupling **(****Figure 4G**).

The steps above are repeated for coupling TNF alpha to site-specific spots different from those coupled to IL-6 using a different reticle to expose a different spot **(****Figure 5A-5C**). These steps can be repeated several times to couple selected polypeptides to specific spots on an array.

To test binding of IL-6 and TNF alpha to the array, anti-TNF alpha and anti-IL-6 antibodies are added with a dilution of 1:1000 and mixed together in a PBST buffer. All antibodies and buffer solutions are obtained from Life Technologies. The assay was performed as follows: Chips were washed in PBST buffer thrice for 5 minutes. Next, the antibodies were added and incubated for 1 hour at 37°C in the dark. Next, the chips were washed with PBST buffer thrice for 5 minutes followed by deionized water thrice for 5 minutes. Finally, the chips were scanned in a fluorescent scanner.

Fluorescence signal intensity for IL-6 is measured to be 45000 and fluorescence signal intensity for TNF alpha is measured to be 43500 compared to fluorescence signal intensity of no protein at 1500 **(****Figure 7**). This result proves that coupling of two or more proteins can be achieved in an array.

Since the intermediate carbene formed is highly reactive with any X-H bond, this microarray based photoconjugation can be extended to cover small molecules and any chemical or bio molecule that comprises of X-H bond. In the case of benzophenone, photolysis at deep UV causes it to react with C-H bonds. Thus photoconjugation of proteins in a microarray format one at a time can be used to not only generate an array comprising antibodies and other macro biomolecules, but also can be used to develop an array comprising small molecules.

## Claims

1. A method of attaching a polypeptide to a surface, comprising:
obtaining a surface comprising a plurality of free amine groups attached to said surface;
attaching a photoactivatable conjugation compound to said surface by contacting said surface with a conjugation solution comprising said photoactivatable conjugation compound, wherein said photoactivatable conjugation compound comprises an activated carboxylic acid group, and wherein said activated carboxylic acid group binds to the free amine groups attached to said surface;
contacting said surface with a polypeptide; and
selectively exposing said surface to electromagnetic radiation, wherein said electromagnetic radiation activates said attached photoactivatable conjugation compound and wherein said attached activated photoactivatable conjugation compound binds to said polypeptide, thereby attaching said polypeptide to said surface.

2. A method of attaching a polypeptide to a surface, comprising:
obtaining a surface comprising a plurality of free carboxylic acid groups attached to said surface;
contacting said surface with a carboxylic acid activation solution, thereby activating said carboxylic acid groups for binding to an amine group, wherein said carboxylic acid activation solution comprises a carbodiimide or N-hydroxysuccinimide or a compound selected from the group consisting of: 1-ethyl-3-(3-dimethylaminopropyl) carbodiimide, N,N'-diisopropylcarbodiimide, (Benzotriazol-1-yloxy)tripyrrolidinophosphonium hexafluorophosphate, bromo(tripyrroli din-1 - yl)phosphonium hexafluorophosphate, O-(7-azabenzotriazol-1-yl)-N,N,N',N'-tetramethyluronium hexafluorophosphate, O-Benzotriazole-N,N,N',N'-tetramethyl-uronium-hexafluoro-phosphate, and O-(Benzotriazol-1-yl)-N,N,N',N'-tetramethyluronium tetrafluoroborate,
attaching a photoactivatable conjugation compound to said surface by contacting said surface with a conjugation solution comprising said photoactivatable conjugation compound, wherein said photoactivatable conjugation compound comprises an amine group, and wherein said amine group binds to the activated carboxylic acid group attached to said surface;
contacting said surface with a polypeptide; and
selectively exposing said surface to electromagnetic radiation, wherein said electromagnetic radiation activates said attached photoactivatable conjugation compound and wherein said attached activated photoactivatable conjugation compound bind to said polypeptide, thereby attaching said polypeptide to said surface.

3. The method of claim 1 or 2, wherein said method further comprises washing said surface after attaching said photoactivatable conjugation compound to said surface.

4. The method of claim 1 or 2, wherein said activated photoactivatable conjugation compound binds to a polypeptide at a site selected from the group consisting of: a peptide backbone, a side group, an amine group, a carboxylic acid group.

5. The method of claim 1 or 2, wherein said contacting said surface with said conjugation solution comprises spin coating said conjugation solution onto said surface.

6. The method of claim 1 or 2, wherein said electromagnetic radiation has a wavelength of 248 nm; or a wavelength of 330-370 nm.

7. The method of claim 1 or 2, wherein said photoactivatable conjugation compound comprises a moiety selected from the group consisting of: a diazirine moiety, an aryl azide moiety, and a benzophenone moiety.

8. The method of claim 1 or 2, wherein said conjugation solution comprises a polymer, and optionally wherein said polymer is polyvinyl pyrrollidone.

9. The method of claim 2, wherein said method further comprises washing said surface after activating said carboxylic acid groups attached to said surface.

10. The method of claim 2, wherein said photoactivatable conjugation compound comprises an N-hydroxysuccinimide moiety attached to an ester; or an N-hydroxysuccinimide ester functionally attached to a moiety selected from the group consisting of: a diazirine moiety, an aryl azide moiety, and a benzophenone moiety.

11. The method of claim 1 or 2, wherein said polypeptide is a protein.

12. The method of claim 1 or 2, wherein said attachment of said polypeptide at said selectively exposed surfaces has an attachment efficiency of at least 90%, 91%, 92%, 93%, 94%, 95%, 96%, 97%, 98%, or 99%.

13. The method of claim 1 or 2, wherein said attachment of said polypeptides to said array has a high stability.

14. The method of claim 1 or 2, wherein said method is repeated to generate a surface comprising a plurality of unique polypeptides attached to said surface at selected locations.

15. The method of claim 1 or 2, wherein said surface comprises at least 2, optionally at least 10, optionally at least 100, optionally at least 1,000, and optionally at least 10,000, unique polypeptides attached to said surface at selected locations.

## Patentansprüche

1. Verfahren zum Anbringen eines Polypeptids an eine Oberfläche, wobei das Verfahren Folgendes umfasst:
Erhalten einer Oberfläche, die eine Vielzahl von freien Amingruppen umfasst, die an die Oberfläche gebunden sind;
Anbringen einer photoaktivierbaren Konjugationsverbindung an der Oberfläche durch Inkontaktbringen der Oberfläche mit einer Konjugationslösung, die die photoaktivierbare Konjugationsverbindung enthält, wobei die photoaktivierbare Konjugationsverbindung eine aktivierte Carbonsäuregruppe umfasst, und wobei die aktivierte Carbonsäuregruppe an die freien Amingruppen bindet, die an die Oberfläche gebunden sind;
Inkontaktbringen der Oberfläche mit einem Polypeptid; und
selektives Aussetzen der Oberfläche einer elektromagnetischen Strahlung, wobei die elektromagnetische Strahlung die angehängte photoaktivierbare Konjugationsverbindung aktiviert und wobei die angehängte aktivierte photoaktivierbare Konjugationsverbindung an das Polypeptid bindet, wodurch das Polypeptid an die Oberfläche gebunden wird.

2. Verfahren zum Anbringen eines Polypeptids an eine Oberfläche, wobei das Verfahren Folgendes umfasst:
Erhalten einer Oberfläche, die eine Vielzahl von freien Carbonsäuregruppen umfasst, die an die Oberfläche gebunden sind;
Inkontaktbringen der Oberfläche mit einer Carbonsäureaktivierungslösung, wodurch die Carbonsäuregruppen zur Bindung an eine Amingruppe aktiviert werden, wobei die Carbonsäureaktivierungslösung ein Carbodiimid oder N-Hydroxysuccinimid oder eine Verbindung, ausgewählt aus der Gruppe bestehend aus Folgendem umfasst: 1-Ethyl-3-(3-dimethylaminopropyl)carbodiimid, N,N'-Diisopropylcarbodiimid, (Benzotriazol-1-yloxy)tripyrrolidinophosphonium-hexafluorphosphat, Brom(tripyrrolidin-1-yl)phosphonium-hexafluorphosphat, O-(7-Azabenzotriazol-1-yl)-N,N,N',N'-tetramethyluronium-hexafluorphosphat, O-Benzotriazol-N,N,N',N'-tetramethyluronium-hexafluorphosphat und O-(Benzotriazol-1-yl)-N,N,N',N'-Tetramethyluroniumtetrafluorborat,
Anbringen einer photoaktivierbaren Konjugationsverbindung an der Oberfläche durch Inkontaktbringen der Oberfläche mit einer Konjugationslösung, die die photoaktivierbare Konjugationsverbindung enthält, wobei die photoaktivierbare Konjugationsverbindung eine Amingruppe umfasst, und wobei die Amingruppe an die aktivierte Carbonsäuregruppe bindet, die an die Oberfläche gebunden ist;
Inkontaktbringen der Oberfläche mit einem Polypeptid; und
selektives Aussetzen der Oberfläche einer elektromagnetischen Strahlung, wobei die elektromagnetische Strahlung die angehängte photoaktivierbare Konjugationsverbindung aktiviert und wobei die angehängte aktivierte photoaktivierbare Konjugationsverbindung an das Polypeptid bindet, wodurch das Polypeptid an die Oberfläche gebunden wird.

3. Verfahren nach Anspruch 1 oder 2, wobei das Verfahren ferner das Waschen der Oberfläche nach dem Anbringen der photoaktivierbaren Konjugationsverbindung an der Oberfläche umfasst.

4. Verfahren nach Anspruch 1 oder 2, wobei die aktivierte photoaktivierbare Konjugationsverbindung an ein Polypeptid an einer Stelle bindet, die ausgewählt ist aus der Gruppe bestehend aus: einem Peptidrückgrat, einer Seitengruppe, einer Amingruppe, einer Carbonsäuregruppe.

5. Verfahren nach Anspruch 1 oder 2, wobei das Inkontaktbringen der Oberfläche mit der Konjugationslösung das Aufschleudern der Konjugationslösung auf die Oberfläche umfasst.

6. Verfahren nach Anspruch 1 oder 2, wobei die elektromagnetische Strahlung eine Wellenlänge von 248 nm; oder eine Wellenlänge von 330 bis 370 nm aufweist.

7. Verfahren nach Anspruch 1 oder 2, wobei die photoaktivierbare Konjugationsverbindung eine Einheit umfasst, die ausgewählt ist aus der Gruppe bestehend aus: einer Diazirin-Einheit, einer Arylazid-Einheit und einer Benzophenon-Einheit.

8. Verfahren nach Anspruch 1 oder 2, wobei die Konjugationslösung ein Polymer umfasst, und optional wobei das Polymer Polyvinylpyrrolidon ist.

9. Verfahren nach Anspruch 2, wobei das Verfahren ferner das Waschen der Oberfläche nach dem Aktivieren der an die Oberfläche gebundenen Carbonsäuregruppen umfasst.

10. Verfahren nach Anspruch 2, wobei die photoaktivierbare Konjugationsverbindung Folgendes umfasst: eine N-Hydroxysuccinimid-Einheit, die an einen Ester gebunden ist, oder einen N-Hydroxysuccinimid-Ester, der funktionell an eine Einheit gebunden ist, die aus der Gruppe ausgewählt ist, die aus Folgendem besteht: einer Diazirinkomponente, einer Arylazidkomponente und einer Benzophenonkomponente.

11. Verfahren nach Anspruch 1 oder 2, wobei das Polypeptid ein Protein ist.

12. Verfahren nach Anspruch 1 oder 2, wobei die Bindung des Polypeptids an den selektiv exponierten Oberflächen eine Bindungseffizienz von mindestens 90%, 91%, 92%, 93%, 94%, 95%, 96%, 97%, 98% oder 99% aufweist.

13. Verfahren nach Anspruch 1 oder 2, wobei die Bindung der Polypeptide an das Array eine hohe Stabilität aufweist.

14. Verfahren nach Anspruch 1 oder 2, wobei das Verfahren optional wiederholt wird, um eine Oberfläche zu erzeugen, die eine Vielzahl von einzigartigen Polypeptiden umfasst, die an ausgewählten Stellen an die Oberfläche gebunden sind.

15. Verfahren nach Anspruch 1 oder 2, wobei die Oberfläche mindestens 2, optional mindestens 10, optional mindestens 100, optional mindestens 1.000 und optional mindestens 10.000 einzigartige Polypeptide umfasst, die an ausgewählten Stellen an die Oberfläche gebunden sind.

## Revendications

1. Procédé de fixation d'un polypeptide sur une surface, comprenant :
l'obtention d'une surface comprenant une pluralité de groupes amine libres fixés sur ladite surface ;
la fixation d'un composé de conjugaison photoactivable à ladite surface en mettant en contact ladite surface avec une solution de conjugaison comprenant ledit composé de conjugaison photoactivable, dans lequel ledit composé de conjugaison photoactivable comprend un groupe acide carboxylique activé, et dans lequel ledit groupe acide carboxylique activé se lie aux groupes amine libres fixés à ladite surface ;
la mise de ladite surface en contact avec un polypeptide ; et
l'exposition sélective de ladite surface à un rayonnement électromagnétique, dans lequel ledit rayonnement électromagnétique active ledit composé de conjugaison photoactivable fixé et dans lequel ledit composé de conjugaison photoactivable activé fixé se lie audit polypeptide, fixant ainsi ledit polypeptide à ladite surface.

2. Procédé de fixation d'un polypeptide à une surface, comprenant :
l'obtention d'une surface comprenant une pluralité de groupes acide carboxylique libres fixés à ladite surface ;
la mise en contact de ladite surface avec une solution d'activation d'acide carboxylique, activant ainsi lesdits groupes acide carboxylique pour la liaison à un groupe amine, dans lequel ladite solution d'activation d'acide carboxylique comprend un carbodiimide ou N-hydroxysuccinimide ou un composé choisi dans le groupe constitué par : 1-éthyl-3-(3-diméthylaminopropyl) carbodiimide, N,N'-diisopropylcarbodiimide, (Benzotriazol-1-yloxy)tripyrrolidinophosphonium hexafluorophosphate, bromo(tripyrrolidin-1-yl)phosphonium hexafluorophosphate, O-(7-azabenzotriazol-1-yl)-N,N,N' ,N' -tétraméthyluronium hexafluorophosphate, O-benzotriazole-N,N,N' ,N' -tétraméthyl-uronium-hexafluoro-phosphate et O-(benzotriazole-1-yl)-N,N,N' ,N' - tétraméthyluronium tétrafluoroborate,
la fixation d'un composé de conjugaison photoactivable à ladite surface en mettant en contact ladite surface avec une solution de conjugaison comprenant ledit composé de conjugaison photoactivable, dans lequel ledit composé de conjugaison photoactivable comprend un groupe amine, et dans lequel ledit groupe amine se lie au groupe acide carboxylique activé fixé à ladite surface ;
la mise en contact de ladite surface avec un polypeptide ; et
l'exposition sélective de ladite surface à un rayonnement électromagnétique, dans lequel ledit rayonnement électromagnétique active ledit composé de conjugaison photoactivable fixé et dans lequel ledit composé de conjugaison photoactivable fixé se lie audit polypeptide, fixant ainsi ledit polypeptide à ladite surface.

3. Procédé selon la revendication 1 ou 2, dans lequel ledit procédé comprend en outre le lavage de ladite surface après fixation dudit composé de conjugaison photoactivable à ladite surface.

4. Procédé selon la revendication 1 ou 2, dans lequel ledit composé de conjugaison photoactivable activé se lie à un polypeptide en un site choisi dans le groupe constitué par : un squelette peptidique, un groupe latéral, un groupe amine, un groupe acide carboxylique.

5. Procédé selon la revendication 1 ou 2, dans lequel ladite mise en contact de ladite surface avec ladite solution de conjugaison comprend le revêtement par centrifugation de ladite solution de conjugaison sur ladite surface.

6. Procédé selon la revendication 1 ou 2, dans lequel ledit rayonnement électromagnétique a une longueur d'onde de 248 nm ; ou une longueur d'onde de 330 à 370 nm.

7. Procédé selon la revendication 1 ou 2, dans lequel ledit composé de conjugaison photoactivable comprend un fragment choisi dans le groupe constitué par : un fragment diazirine, un fragment aryl azide et un fragment benzophénone.

8. Procédé selon la revendication 1 ou 2, dans lequel ladite solution de conjugaison comprend un polymère, et éventuellement dans lequel ledit polymère est la polyvinyl pyrrollidone.

9. Procédé selon la revendication 2, dans lequel ledit procédé comprend en outre le lavage de ladite surface après activation desdits groupes acide carboxylique fixés à ladite surface.

10. Procédé selon la revendication 2, dans lequel ledit composé de conjugaison photoactivable comprend un fragment N-hydroxysuccinimide fixé à un ester ; ou un ester de N-hydroxysuccinimide lié de manière fonctionnelle à un fragment choisi dans le groupe constitué par : un fragment diazirine, un fragment aryl azide et un fragment benzophénone.

11. Procédé selon la revendication 1 ou 2, dans lequel ledit polypeptide est une protéine.

12. Procédé selon la revendication 1 ou 2, dans lequel ladite fixation dudit polypeptide au niveau desdites surfaces sélectivement exposées a une efficacité de fixation d'au moins 90%, 91%, 92%, 93%, 94%, 95%, 96%, 97%, 98% ou 99%.

13. Procédé selon la revendication 1 ou 2, dans lequel ladite fixation desdits polypeptides audit réseau a une grande stabilité.

14. Procédé selon la revendication 1 ou 2, dans lequel ledit procédé est répété pour générer une surface comprenant une pluralité de polypeptides uniques fixés à ladite surface à des emplacements sélectionnés.

15. Procédé selon la revendication 1 ou 2, dans lequel ladite surface comprend au moins 2, éventuellement au moins 10, éventuellement au moins 100, éventuellement au moins 1000 et éventuellement au moins 10000 polypeptides uniques fixés à ladite surface à des emplacements sélectionnés.
